(19) Europäisches Patentamt
European Patent Office
Office européen des brevets

(11) Veröffentlichungsnummer: **0 212 353 A2**

(12) **EUROPÄISCHE PATENTANMELDUNG**

(21) Anmeldenummer: **86110411.5**

(22) Anmeldetag: **28.07.86**

(51) Int. Cl.⁴: **C 07 D 231/38**, C 07 D 231/16, C 07 D 401/04, A 01 N 43/56

(30) Priorität: **08.08.85 DE 3528478**

(43) Veröffentlichungstag der Anmeldung: **04.03.87 Patentblatt 87/10**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Gehring, Reinhold, Dr., Dasnoeckel 49, D-5600 Wuppertal 11 (DE)**
Erfinder: **Schallner, Otto, Dr., Noldeweg 22, D-4019 Monheim (DE)**
Erfinder: **Stetter, Jörg, Dr., Gellertweg 4, D-5600 Wuppertal (DE)**
Erfinder: **Santel, Hans-Joachim, Dr., Gruenstrasse 9a, D-5090 Leverkusen (DE)**
Erfinder: **Schmidt, Robert R., Dr., Im Waldwinkel 110, D-5060 Bergisch-Gladbach 2 (DE)**

(54) **1-Aryl-5-hydrazino-pyrazole.**

(57) Die Erfindung betrifft neue 1-Aryl-5-hydrazino-pyrazole der allgemeinen Formel (I),

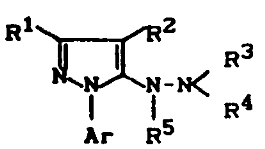

(I)

in welcher
$R^1$ für Wasserstoff oder Alkyl steht,
$R^2$ für Cyano oder Nitro steht,
$R^3$ für Wasserstoff, für jeweils gegebenenfalls substituiertes Alkyl, Alkenyl, Alkinyl oder Cycloalkyl, für einen Rest

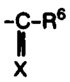

oder für einen Rest $-SO_2-R^7$ steht,
$R^4$ für Wasserstoff, für jeweils gegebenenfalls substituiertes Alkyl, Alkenyl, Alkinyl oder Cycloalkyl, für einen Rest

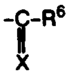

oder für einen Rest $-SO_2-R^7$ steht,
$R^5$ für Wasserstoff steht oder für den Fall, daß $R^4$ für Wasserstoff steht, auch für einen Rest

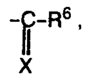,

für einen Rest $-SO_2-R^7$,
oder für jeweils gegebenenfalls substituiertes Alkyl, Alkenyl, Alkinyl oder Cycloalkyl steht, wobei jeweils
X für Sauerstoff oder Schwefel steht,
$R^6$ für Wasserstoff, Alkyl, Alkoxy, Alkoxyalkyl, Alkylthio, Alkylthioalkyl, Alkylamino, Dialkylamino, Halogenalkyl, Alkenyl, Alkinyl, für gegebenenfalls substituiertes Cycloalkyl oder für jeweils gegebenenfalls substituiertes Aryl, Aryloxy, Arylthio oder Arylamino steht und
$R^7$ für Alkyl, Hydroxyalkyl, Alkoxyalkyl, Halogenalkyl, Alkenyl, Alkinyl, Cycloalkyl oder für jeweils gegebenenfalls substituiertes Aralkyl oder Aryl steht und
Ar für substituiertes Phenyl oder für gegebenenfalls substituiertes Pyridyl steht,
mehrere Verfahren zu ihrer Herstellung und ihre Verwendung als Herbizide.

EP 0 212 353 A2

0212353

BAYER AKTIENGESELLSCHAFT 5090 Leverkusen, Bayerwerk
Konzernverwaltung RP
Patentabteilung KM/Ke-c
Ib

1-Aryl-5-hydrazino-pyrazole

Die Erfindung betrifft neue 1-Aryl-5-hydrazino-pyrazole, mehrere Verfahren zu ihrer Herstellung und ihre Verwendung als Herbizide.

Es ist bereits bekannt, daß bestimmte 1-Aryl-pyrazole wie beispielsweise das 4-Cyano-5-propionamido-1-(2,4,6-trichlorphenyl)-pyrazol herbizide Eigenschaften besitzen (vgl. z.B. DE-OS 3 226 513).

Die herbizide Wirksamkeit dieser vorbekannten Verbindungen gegenüber Schadpflanzen ist jedoch ebenso wie ihre Verträglichkeit gegenüber wichtigen Kulturpflanzen nicht immer in allen Anwendungsgebieten völlig zufriedenstellend.

Weiterhin bekannt sind Verbindungen wie beispielsweise 3-Methyl-4-nitro-1-phenyl-5-hydrazin-pyrazol (J. Het. Chem. 20, 277-279 (1983)). Eine Anwendung dieser Verbindungen, insbesondere in der Landwirtschaft, ist jedoch nicht angegeben.

Es wurden nun neue 1-Aryl-5-hydrazino-pyrazole der allgemeinen Formel (I),

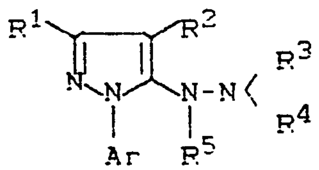

(I)

in welcher

$R^1$ für Wasserstoff oder Alkyl steht,

$R^2$ für Cyano oder Nitro steht,

$R^3$ für Wasserstoff, für jeweils gegebenenfalls substituiertes Alkyl, Alkenyl, Alkinyl oder Cycloalkyl, für einen Rest $-\underset{\underset{X}{\|}}{C}-R^6$ oder für einen Rest $-SO_2-R^7$ steht,

$R^4$ für Wasserstoff, für jeweils gegebenenfalls substituiertes Alkyl, Alkenyl, Alkinyl oder Cycloalkyl, für einen Rest $-\underset{\underset{X}{\|}}{C}-R^6$ oder für einen Rest $-SO_2-R^7$ steht,

$R^5$ für Wasserstoff steht;

oder für den Fall, daß $R^4$ für Wasserstoff steht, auch für einen Rest $-\underset{\underset{X}{\|}}{C}-R^6$, oder für einen Rest

$-SO_2-R^7$ oder für jeweils gegebenenfalls substituiertes Alkyl, Alkenyl, Alkinyl oder Cycloalkyl steht,

wobei jeweils

X für Sauerstoff oder Schwefel steht,

$R^6$ für Wasserstoff, Alkyl, Alkoxy, Alkoxyalkyl, Alkylthio, Alkylthioalkyl, Alkylamino, Dialkylamino, Halogenalkyl, Alkenyl, Alkinyl, für gegebenenfalls substituiertes Cycloalkyl oder für jeweils gegebenenfalls substituiertes Aryl, Aryloxy, Arylthio oder Arylamino steht und

$R^7$ für Alkyl, Hydroxyalkyl, Alkoxyalkyl Halogenalkyl, Alkenyl, Alkinyl, Cycloalkyl oder für jeweils gegebenenfalls substituiertes Aralkyl oder Aryl steht und

Ar für substituiertes Phenyl oder für gegebenenfalls substituiertes Pyridyl steht,

gefunden.

Weiterhin wurde gefunden, daß man die neuen 1-Aryl-5-hydrazino-pyrazole der allgemeinen Formel (I),

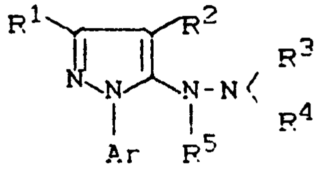

(I)

in welcher

$R^1$ für Wasserstoff oder Alkyl steht,

$R^2$ für Cyano oder Nitro steht,

$R^3$ für Wasserstoff, für jeweils gegebenenfalls substituiertes Alkyl, Alkenyl, Alkinyl oder Cycloalkyl, für einen Rest $-\underset{\underset{X}{\|}}{C}-R^6$ oder für einen Rest $-SO_2-R^7$ steht,

$R^4$ für Wasserstoff, für jeweils gegebenenfalls substituiertes Alkyl, Alkenyl, Alkinyl oder Cycloalkyl, für einen Rest $-\underset{\underset{X}{\|}}{C}-R^6$ oder für einen Rest $-SO_2-R^7$ steht,

$R^5$ für Wasserstoff steht;

oder für den Fall, daß $R^4$ für Wasserstoff steht, auch für einen Rest $-\underset{\underset{X}{\|}}{C}-R^6$, oder für einen Rest $-SO_2-R^7$ oder für jeweils gegebenenfalls substituiertes Alkyl, Alkenyl, Alkinyl oder Cycloalkyl steht,

wobei jeweils

0212353

X für Sauerstoff oder Schwefel steht,

$R^6$ für Wasserstoff, Alkyl, Alkoxy, Alkoxyalkyl, Alkylthio, Alkylthioalkyl, Alkylamino, Dialkylamino, Halogenalkyl, Alkenyl, Alkinyl, für gegebenenfalls substituiertes Cycloalkyl, für jeweils gegebenenfalls substituiertes Aryl, Aryloxy, Arylthio oder Arylamino steht und

$R^7$ für Alkyl, Hydroxyalkyl, Alkoxyalkyl, Halogenalkyl, Alkenyl, Alkinyl, Cycloalkyl oder für jeweils gegebenenfalls substituiertes Aralkyl oder Aryl steht und

Ar für substituiertes Phenyl oder für gegebenenfalls substituiertes Pyridyl steht,

erhält, wenn man

(a) 1-Aryl-5-halogen-pyrazole der Formel (II)

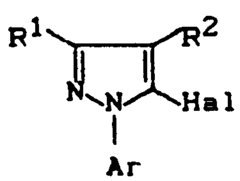

(II)

in welcher

$R^1$, $R^2$ und Ar die oben angegebene Bedeutung haben und

Hal für Halogen steht,

mit Hydrazin-Derivaten der Formel (III)

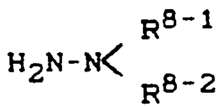 (III)

in welcher

$R^{8-1}$ und $R^{8-2}$ unabhängig voneinander jeweils für Wasserstoff oder für jeweils gegebenenfalls substituiertes Alkyl, Alkenyl, Alkinyl oder Cycloalkyl stehen,

gegebenenfalls in Gegenwart eines Verdünnungsmittels umsetzt, oder wenn man

(b) die nach Verfahren (a) erhältlichen 5-Hydrazino-1-aryl-pyrazole der Formel (Ia),

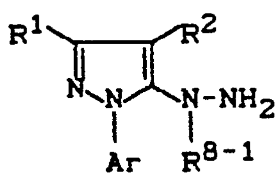 (Ia)

in welcher

$R^1$, $R^2$, $R^{8-1}$ und Ar die oben angegebene Bedeutung haben,

oder der Formel (Ib),

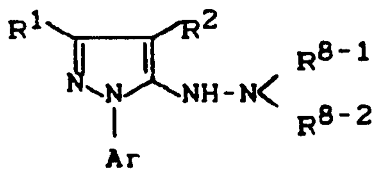

(Ib)

in welcher

$R^1$, $R^2$, $R^{8-1}$, $R^{8-2}$ und Ar die oben angegebene Bedeutung haben,

(α) mit Alkylierungsmitteln der Formel (IV),

$$R^{8-3}\text{-}A^1 \qquad \text{(IV)}$$

in welcher

$R^{8-3}$ für jeweils gegebenenfalls substituiertes Alkyl, Alkenyl, Alkinyl oder Cycloalkyl steht und

$A^1$ für eine elektronenanziehende Abgangsgruppe steht, oder

(β) mit Acylierungsmitteln der Formel (V),

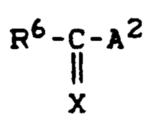

(V)

in welcher

0212353

$R^6$ und X die oben angegebene Bedeutung haben und

$A^2$ für eine elektronenanziehende Abgangsgruppe steht,

oder

(γ) mit Iso(thio)cyanaten der Formel (VI)

$$R^{6-1}\text{-N=C=X} \qquad \text{(VI)}$$

in welcher

$R^{6-1}$ für Alkyl oder für gegebenenfalls substituiertes Aryl steht und

X die oben angegebene Bedeutung hat,

oder

(δ) mit Sulfonylierungsmitteln der Formel (VII),

$$R^7\text{-}SO_2\text{-}A^3 \qquad \text{(VII)}$$

in welcher

$R^7$ die oben angegebene Bedeutung hat und

$A^3$ für eine elektronenanziehende Abgangsgruppe steht,

jeweils gegebenenfalls in Gegenwart eines Verdünnungsmittels sowie gegebenenfalls in Gegenwart eines Säurebindemittels oder Reaktionshilfsmittels umsetzt.

Schließlich wurde gefunden, daß die neuen 1-Aryl-5-hydrazino-pyrazole der Formel (I) herbizide Eigenschaften besitzen.

Überraschenderweise zeigen die erfindungsgemäßen 1-Aryl-5-hydrazino-pyrazole der Formel (I) eine bessere herbizide Wirksamkeit gegenüber Schadpflanzen, bei gleichzeitiger besserer Verträglichkeit gegenüber wichtigen Nutzpflanzen, als beispielsweise die aus dem Stand der Technik bekannte Verbindung 4-Cyano-5-propionamido-1-(2,4,6-trichlorphenyl)-pyrazol, welches eine chemisch und wirkungsmäßig naheliegende Verbindung ist.

Die erfindungsgemäßen 1-Aryl-5-hydrazino-pyrazole sind durch die Formel (I) allgemein definiert. Bevorzugt sind Verbindungen der Formel (I), bei welcher

$R^1$ für Wasserstoff oder für geradkettiges oder verzweigtes Alkyl mit 1 bis 4 Kohlenstoffatomen steht,

$R^2$ für Cyano oder Nitro steht,

$R^3$ für Wasserstoff, für jeweils gegebenenfalls einfach oder mehrfach, gleich oder verschieden substituiertes Alkyl, Alkenyl oder Alkinyl mit jeweils bis zu

8 Kohlenstoffatomen steht, wobei als Substituenten jeweils in Frage kommen: Halogen, Cyano, Nitro, Hydroxy, Carboxy, jeweils geradkettiges oder verzweigtes Alkoxy oder Alkoxycarbonyl mit jeweils 1 bis 6 Kohlenstoffatomen im Alkylteil, oder gegebenenfalls durch Alkyl, Alkoxy, Alkylsulfonyl, Alkenyl oder Alkinyl mit jeweils bis zu 4 Kohlenstoffatomen substituiertes Aminocarbonyl, wobei das Stickstoffatom des Aminocarbonylrestes auch Teil eines gesättigten 3- bis 7-gliedrigen Heterocyclus sein kann, der gegebenenfalls auch 1 oder 2 weitere Heteroatome, insbesondere Stickstoff, Sauerstoff oder Schwefel, enthalten kann; außerdem für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen steht, für einen Rest $-\underset{\underset{X}{\|}}{C}-R^6$

oder für einen Rest $SO_2-R^7$ steht,

$R^4$ für Wasserstoff, für jeweils gegebenenfalls einfach oder mehrfach, gleich oder verschieden substituiertes Alkyl, Alkenyl oder Alkinyl mit jeweils bis zu 8 Kohlenstoffatomen steht, wobei als Substituenten jeweils in Frage kommen: Halogen, Cyano, Nitro, Hydroxy, Carboxy, jeweils geradkettiges oder verzweigtes Alkoxy oder Alkoxycarbonyl mit jeweils 1 bis 6 Kohlenstoffatomen im Alkylteil, oder gegebenenfalls durch Alkyl, Alkoxy, Alkylsulfonyl, Alkenyl oder Alkinyl mit jeweils bis zu 4 Kohlenstoffatomen substituiertes Aminocarbonyl, wobei das Stickstoff

atom des Aminocarbonylrestes auch Teil eines gesättigten 3- bis 7-gliedrigen Heterocyclus sein kann, der gegebenenfalls auch 1 oder 2 weitere Heteroatome, insbesondere Stickstoff, Sauerstoff oder Schwefel, enthalten kann;
außerdem für gegebenenfalls einfach oder mehrfach, gleich oder verschieden durch Halogen und geradkettiges oder verzweigtes Alkyl mit 1 bis 4 Kohlenstoffatomen substituiertes Cycloalkyl mit 3 bis 7 Kohlenstoffatomen steht, für einen Rest $-\underset{\underset{X}{\|}}{C}-R^6$

oder für einen Rest $SO_2-R^7$ steht,

$R^5$ für Wasserstoff steht;
oder für den Fall, daß $R^4$ für Wasserstoff steht, auch für einen Rest $-\underset{\underset{X}{\|}}{C}-R^6$ ,für einen

Rest $-SO_2-R^7$ steht oder für jeweils gegebenenfalls einfach oder mehrfach, gleich oder verschieden substituiertes Alkyl, Alkenyl oder Alkinyl mit jeweils bis zu 8 Kohlenstoffatomen steht, wobei als Substituenten jeweils in Frage kommen: Halogen, Cyano, Nitro, Hydroxy, Carboxy, jeweils geradkettiges oder verzweigtes Alkoxy oder Alkoxycarbonyl mit jeweils bis zu 6 Kohlenstoffatomen im Alkylteil, oder gegebenenfalls durch Alkyl, Alkoxy, Alkylsulfonyl, Alkenyl oder Alkinyl mit jeweils bis zu 4 Kohlenstoffatomen substituiertes Aminocarbonyl, wobei das Stickstoffatom des Aminocarbonylrestes auch Teil eines gesättigten 3- bis 7-gliedrigen

Heterocyclus sein kann, der gegebenenfalls 1 oder 2 weitere Heteroatome, insbesondere Stickstoff, Sauerstoff oder Schwefel, enthalten kann; und außerdem für gegebenenfalls einfach oder mehrfach, gleich oder verschieden durch Halogen und geradkettiges oder verzweigtes Alkyl mit 1 bis 4 Kohlenstoffatomen substituiertes Cycloalkyl mit 3 bis 7 Kohlenstoffatomen steht,

wobei jeweils

X für Sauerstoff oder Schwefel steht,

$R^6$ für Wasserstoff, für jeweils geradkettiges oder verzweigtes Alkyl, Alkoxy, Alkoxyalkyl, Alkylthio, Alkylthioalkyl, Alkylamino oder Dialkylamino mit jeweils 1 bis 4 Kohlenstoffatomen in den einzelnen Alkylteilen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkenyl oder Alkinyl mit 2 bis 4 Kohlenstoffatomen, für gegebenenfalls einfach oder mehrfach, gleich oder verschieden durch Halogen, jeweils geradkettiges oder verzweigtes Alkyl mit 1 bis 4 Kohlenstoffatomen oder Halogenalkyl mit 1 bis 4 Kohlenstoffatomen und 1 bis 9 gleichen oder verschiedenen Halogenatomen substituiertes Cycloalkyl mit 3 bis 7 Kohlenstoffatomen oder für jeweils gegebenenfalls einfach oder mehrfach, gleich oder verschieden substituiertes Phenyl,

Phenoxy, Phenylthio oder Phenylamino steht, wobei als Phenylsubstituenten jeweils ausgewählt sind: Halogen, jeweils geradkettiges oder verzweigtes Alkyl, Alkoxy mit jeweils 1 bis 4 Kohlenstoffatomen oder Halogenalkyl mit 1 bis 4 Kohlenstoffatomen und 1 bis 9 gleichen oder verschiedenen Halogenatomen, und

$R^7$ für jeweils geradkettiges oder verzweigtes Alkyl, Hydroxyalkyl oder Alkoxyalkyl mit jeweils 1 bis 4 Kohlenstoffatomen in den einzelnen Alkylteilen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkenyl oder Alkinyl mit jeweils 2 bis 4 Kohlenstoffatomen, für Cycloalkyl mit 3 bis 7 Kohlenstoffatomen oder für jeweils gegebenenfalls einfach oder mehrfach, gleich oder verschieden durch Halogen, Cyano, Nitro jeweils geradkettiges oder verzweigtes Alkyl, Alkoxy oder Alkylthio mit jeweils 1 bis 4 Kohlenstoffatomen oder Halogenalkyl mit 1 bis 4 Kohlenstoffatomen und 1 bis 9 gleichen oder verschiedenen Halogenatomen substituiertes Benzyl oder Phenyl steht und

Ar für einfach oder mehrfach, gleich oder verschieden substituiertes Phenyl oder für jeweils gegebenenfalls einfach oder mehrfach, gleich oder verschieden substituiertes 2-Pyridyl, 3-Pyridyl oder 4-Pyridyl steht, wobei als Substituenten jeweils in Frage kommen: Cyano, Nitro,

Halogen, jeweils geradkettiges oder verzweigtes Alkyl, Alkoxy oder Alkoxycarbonyl mit jeweils 1 bis 4 Kohlenstoffatomen im Alkylteil, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^9$, wobei

$R^9$ für Amino, für jeweils geradkettiges oder verzweigtes Alkyl, Alkylamino oder Dialkylamino mit jeweils 1 bis 4 Kohlenstoffatomen in den einzelnen Alkylteilen oder Halogenalkyl mit 1 bis 4 Kohlenstoffatomen und 1 bis 9 gleichen oder verschiedenen Halogenatomen steht und

m für eine Zahl 0, 1 oder 2 steht.

Besonders bevorzugt sind 1-Aryl-5-hydrazino-pyrazole der allgemeinen Formel (I), bei welchen

$R^1$ für Wasserstoff, Methyl, Ethyl, n- oder i-Propyl, n-, i-, s- oder t-Butyl steht,

$R^2$ für Cyano oder Nitro steht,

$R^3$ für Wasserstoff, für jeweils gegebenenfalls ein- bis dreifach, gleich oder verschieden substituiertes Methyl, Ethyl, n- oder i-Propyl, n-, i-, s- oder t-Butyl, Allyl, Butenyl, Propargyl oder Butinyl steht, wobei als Substituenten jeweils in Frage kommen: Fluor, Chlor, Brom, Iod, Cyano, Nitro, Hydroxy,

Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N-Methyl- N-ethylaminocarbonyl, N-Methyl-N-propylaminocarbonyl, N-Ethyl-N-propylaminocarbonyl, N-Methyl-N-methoxyaminocarbonyl, N,N-Diallylaminocarbonyl, N-Methyl-N-propargylaminocarbonyl, N-Methylsulfonylaminocarbonyl, N-Ethylsulfonylaminocarbonyl, Pyrrolidin-1-ylcarbonyl, Piperidin-1-ylcarbonyl, Morpholin-4-ylcarbonyl oder Perhydroazepin-1-ylcarbonyl; außerdem für jeweils gegebenenfalls ein- bis fünffach, gleich oder verschieden durch Chlor, Methyl oder Ethyl substituiertes Cyclopropyl, Cyclopentyl, Cyclohexyl oder Cycloheptyl steht oder für einen Rest-$\underset{\underset{X}{\|}}{C}-R^6$

oder für einen Rest-$SO_2-R^7$ steht,

$R^4$ für Wasserstoff, für jeweils gegebenenfalls ein- bis dreifach, gleich oder verschieden substituiertes Methyl, Ethyl, n- oder i-Propyl, n-, i-, s- oder t-Butyl, Allyl, Butenyl, Propargyl oder Butinyl steht, wobei als Substituenten jeweils in Frage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N-Methyl- N-ethylaminocarbonyl, N-Methyl-N-propylaminocarbonyl, N-Ethyl-N-propylaminocarbonyl, N-Methyl-N-methoxyaminocarbonyl, N,N-Diallylaminocarbonyl, N-Methyl-N-propargylaminocarbonyl, N-Methylsulfonylaminocarbonyl, N-Ethylsulfonylaminocarbonyl, Pyrrolidin-1-ylcarbonyl, Piperidin-1-ylcarbonyl, Morpholin-4-ylcarbonyl oder Perhydroazepin-1-ylcarbonyl; außerdem für jeweils gegebenenfalls ein- bis fünffach, gleich oder verschieden durch Chlor, Methyl oder Ethyl substituiertes Cyclopropyl, Cyclopentyl, Cyclohexyl oder Cycloheptyl steht und darüberhinaus für einen Rest $-\underset{X}{\underset{\|}{C}}-R^6$ oder für einen Rest $-SO_2-R^7$ steht,

$R^5$ für Wasserstoff steht;

oder für den Fall, daß $R^4$ für Wasserstoff steht, auch für einen Rest $-\overset{X}{\overset{\|}{C}}-R^6$, für einen Rest $-SO_2-R^7$ oder für jeweils gegebenenfalls ein- bis dreifach, gleich oder verschieden substituiertes Methyl, Ethyl, n- oder i-Propyl, n-, i-, s- oder t-Butyl, Allyl, Butenyl, Propargyl oder Butinyl steht, wobei als Substituenten jeweils in Frage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl,

N-Methylaminocarbonyl, N-Ethylaminocarbonyl, N,N-Dimethylaminocarbonyl, N,N-Diethylaminocarbonyl, N-Methyl-N-ethylaminocarbonyl, N-Methyl-N-propylaminocarbonyl, N-Ethyl-N-propylaminocarbonyl, N-Methyl-N-methoxyaminocarbonyl, N,N-Diallylaminocarbonyl, N-Methyl-N-propargylaminocarbonyl, N-Methylsulfonylaminocarbonyl, N-Ethylsulfonylaminocarbonyl, Pyrrolidin-1-ylcarbonyl, Piperidin-1-ylcarbonyl, Morpholin-4-ylcarbonyl oder Perhydroazepin-1-ylcarbonyl; außerdem für jeweils gegebenenfalls ein- bis fünffach, gleich oder verschieden durch Chlor, Methyl oder Ethyl substituiertes Cyclopropyl, Cyclopentyl, Cyclohexyl oder Cycloheptyl steht,

wobei jeweils

X für Sauerstoff oder Schwefel steht,

$R^6$ für Wasserstoff, Methyl, Ethyl, n- oder i-Propyl, Methoxy, Ethoxy, Methoxymethyl, Ethoxymethyl, Methoxyethyl, Ethoxyethyl, Methylthio, Ethylthio, Methylthiomethyl, Methylamino, Ethylamino, Dimethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, Heptafluor-n-propyl, Allyl, Propargyl, Butenyl, für jeweils gegebenenfalls ein- bis vierfach, gleich oder verschieden durch Fluor, Chlor, Brom, Methyl und Trifluormethyl substituiertes

Cyclopropyl, Cyclopentyl oder Cyclohexyl oder für jeweils gegebenenfalls ein- bis dreifach, gleich oder verschieden durch Chlor, Methyl, Methoxy oder Trifluormethyl substituiertes Phenyl, Phenoxy, Phenylthio oder Phenylamino steht, und

$R^7$ für Methyl, Ethyl, n- oder i-Propyl, n-, i-, s- oder t-Butyl, Hydroxymethyl, Hydroxyethyl, Methoxymethyl, Methoxyethyl, Chlormethyl, Dichlormethyl, Trifluormethyl, Allyl, Butenyl, Cyclopropyl, Cyclopentyl, Cyclohexyl oder für jeweils gegebenenfalls ein- bis dreifach, gleich oder verschieden substituiertes Benzyl oder Phenyl steht, wobei als Substituenten jeweils in Frage kommen: Fluor, Chlor, Brom, Cyano, Nitro, Methyl, Ethyl, Methoxy, Methylthio oder Trifluormethyl,

und

Ar für ein- bis fünffach, gleich oder verschieden substituiertes Phenyl oder für jeweils gegebenenfalls ein- bis vierfach, gleich oder verschieden substituiertes 2-Pyridyl, 3-Pyridyl oder 4-Pyridyl steht, wobei als Substituenten jeweils in Frage kommen: Cyano, Nitro, Fluor, Chlor, Brom, Iod, Methyl, Ethyl, n- oder i-Propyl, n-, i-, s- oder t-Butyl, Methoxy, Ethoxy, Methoxycarbonyl, Ethoxycarbonyl, Trifluormethyl, Trichlormethyl, Difluorchlormethyl, Difluorchlorethyl, Chlormethyl, Dichlormethyl, Difluormethyl, Pentafluorethyl, Tetrafluorethyl, Tri-

0212353

fluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^9$, wobei

$R^9$ für Amino, Methyl, Ethyl, Methylamino, Ethylamino, Dimethylamino, Diethylamino, Fluordichlormethyl, Difluormethyl, Tetrafluorethyl, Trifluorchlorethyl oder Trifluormethyl steht und

m für eine Zahl 0, 1 oder 2 steht.

Im einzelnen seien außer den bei den Herstellungsbeispielen genannten Verbindungen die folgenden 1-Aryl-5-hydrazino-pyrazole der allgemeinen Formel (I) genannt:

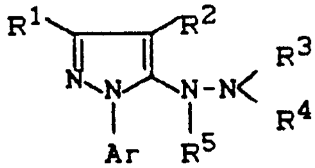

(I)

Tabelle 1

| $R^1$ | $R^2$ | $R^3$ | $R^4$ | $R^5$ | Ar |
|---|---|---|---|---|---|
| H | $NO_2$ | $CH_3$ | $CH_3$ | H | 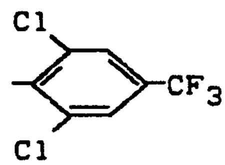 |
| H | CN | H | $-\overset{O}{\overset{\|}{C}}-CH_3$ | H | 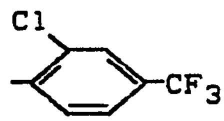 |
| H | CN | H | $-SO_2-CH_3$ | H | 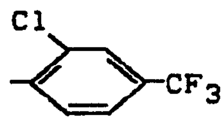 |
| H | CN | H | $-\overset{O}{\overset{\|}{C}}-NH-CH_3$ | H | 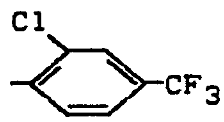 |
| H | CN | H | $-\overset{O}{\overset{\|}{C}}-CH_3$ | $CH_3$ | 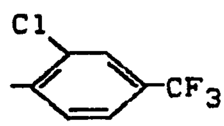 |
| H | CN | H | $-\overset{O}{\overset{\|}{C}}-C_2H_5$ | $CH_3$ | 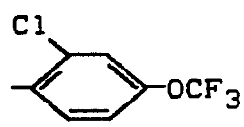 |

Tabelle 1 (Fortsetzung):

| $R^1$ | $R^2$ | $R^3$ | $R^4$ | $R^5$ | Ar |
|---|---|---|---|---|---|
| H | $NO_2$ | H | $CH_3$ | $CH_3$ | 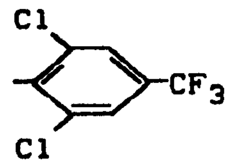 |
| H | $NO_2$ | $CH_3$ | H | H | 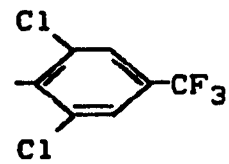 |
| H | $NO_2$ | $CH_3$ | $-\overset{O}{\overset{\|}{C}}-CH_3$ | H | 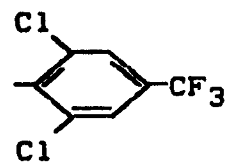 |
| H | $NO_2$ | H | $-SO_2-CH_3$ | H | 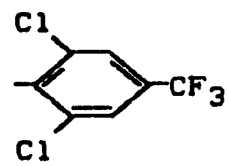 |

Verwendet man beispielsweise 5-Chlor-4-cyano-1-(3,5-dichlor-2-pyridyl)-pyrazol und Hydrazinhydrat als Ausgangsstoffe, so läßt sich der Reaktionsablauf des erfindungsgemäßen Verfahrens (a) durch das folgende Formelschema darstellen:

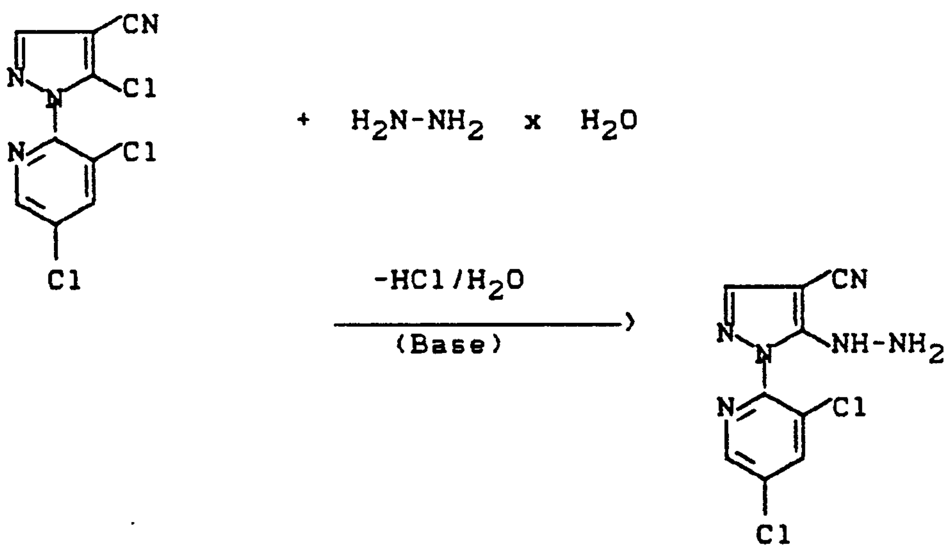


Verwendet man beispielsweise 4-Cyano-5-(α-methyl-hydrazino)-1-(2,4,6-trichlorphenyl)-pyrazol und Bromessigsäureethylester als Ausgangsstoffe, so läßt sich der Reaktionsablauf des erfindungsgemäßen Verfahrens (b-α) durch das folgende Formelschema darstellen:

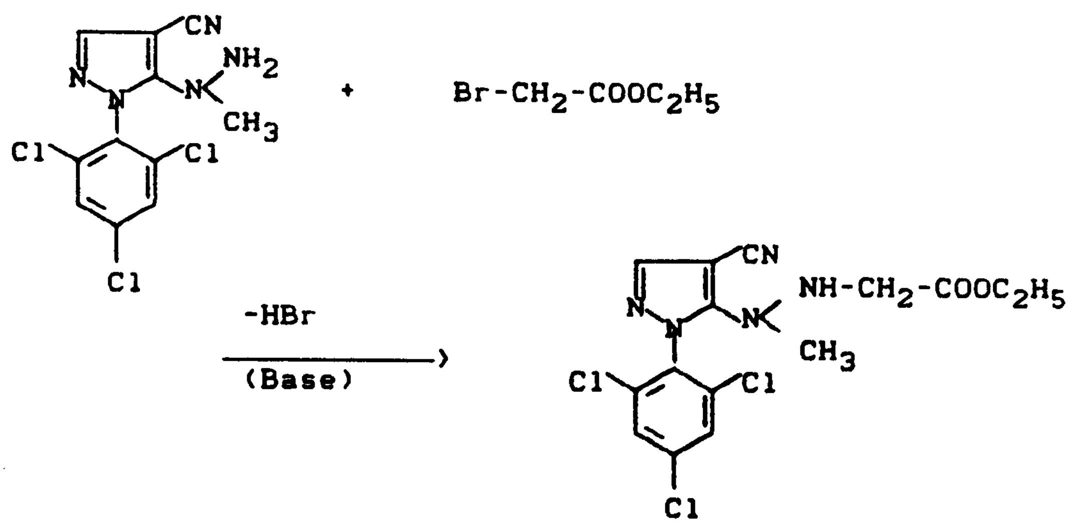


Verwendet man beispielsweise 4-Cyano-5-hydrazino-1-(2,4-dichlorphenyl)-pyrazol und Acetylchlorid als Ausgangsstoffe, so läßt sich der Reaktionsablauf des erfindungsgemäßen Verfahrens (b-β) durch das folgende Formelschema darstellen:

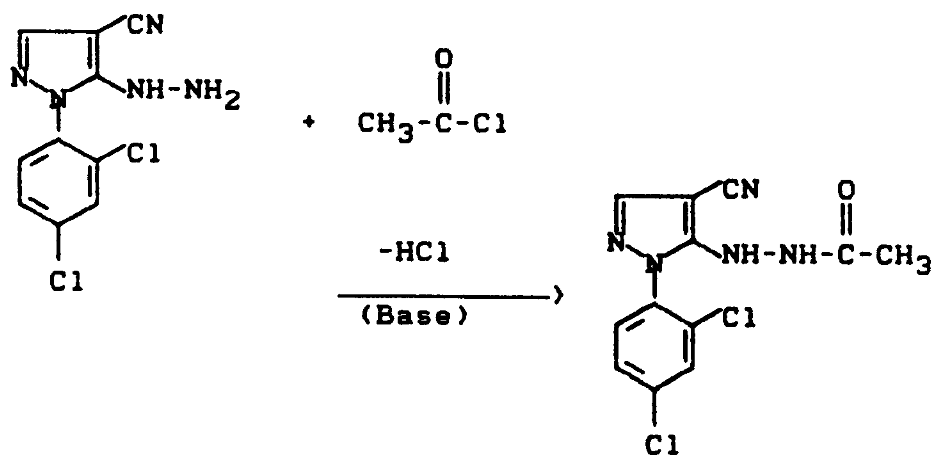

Verwendet man beispielsweise 5-Hydrazino-3-methyl-4-nitro-1-(2,6-dichlor-4-trifluormethyl-phenyl)-pyrazol und Methylisocyanat als Ausgangsstoffe, so läßt sich der Reaktionsablauf des erfindungsgemäßen Verfahrens (b-γ) durch das folgende Formelschema darstellen:

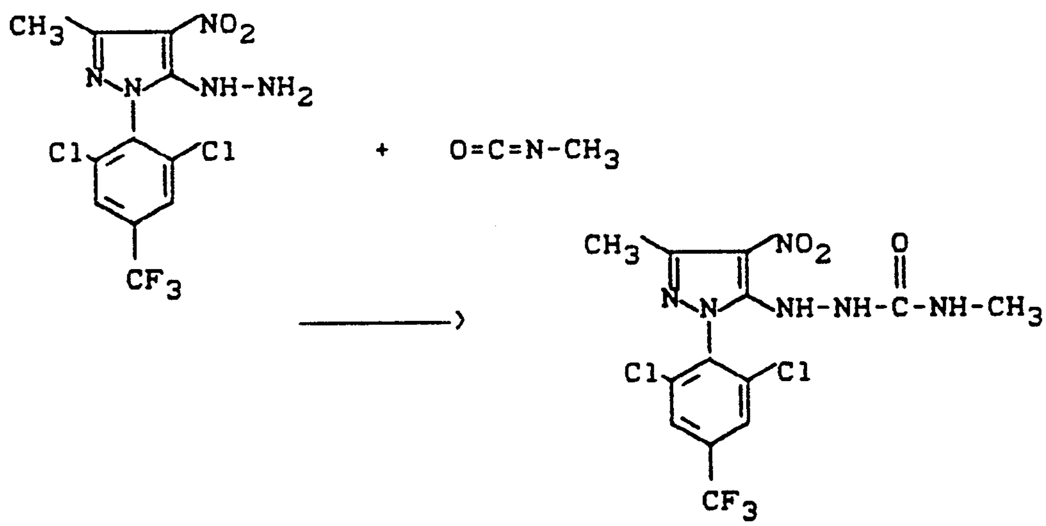

Verwendet man beispielsweise 5-Hydrazino-4-nitro-1-(2-pyridyl)-pyrazol und Methylsulfonsäurechlorid als Ausgangsstoffe, so läßt sich der Reaktionsablauf des erfindungsgemäßen Verfahrens (b-δ) durch das folgende Formelschema darstellen:

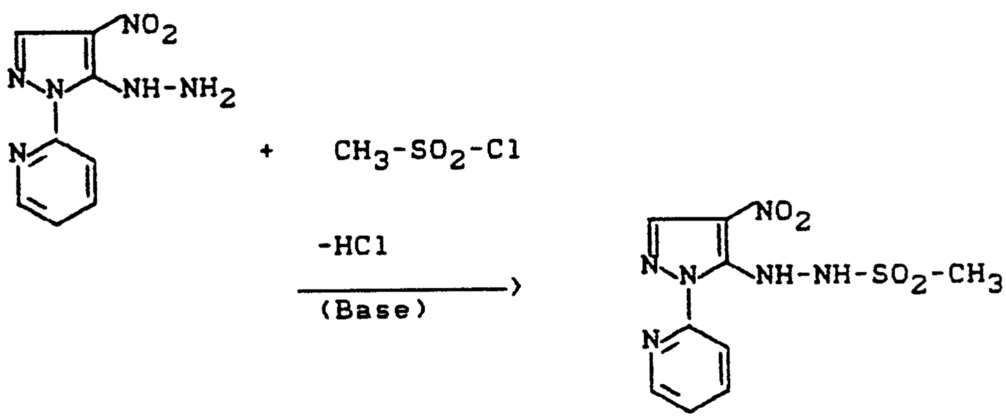

Die zur Durchführung des erfindungsgemäßen Verfahrens (a) als Ausgangsstoffe benötigten 1-Aryl-5-halogen-pyrazole sind durch die Formel (II) allgemein definiert. In dieser Formel (II) stehen $R^1$, $R^2$ und Ar vorzugsweise für diejenigen Reste, die bereits im Zusammenhang mit der Beschreibung der erfindungsgemäßen Stoffe der Formel (I) als bevorzugt für diese Substituenten genannt wurden.
Hal steht vorzugsweise für Chlor oder Brom.

Die 1-Aryl-5-halogen-pyrazole der Formel (II) sind noch nicht bekannt. Sie sind jedoch zum Teil Gegenstand eigener vorgängiger Patentanmeldungen (vgl. DE-P 3 501 323 vom 17.01.1985 und DE-P 35 20 329 vom 07.06.1985).

Man erhält sie beispielsweise, wenn man 5-Amino-1-aryl-pyrazole der Formel (VIII),

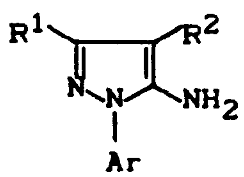

(VIII)

in welcher

$R^1$, $R^2$ und Ar die oben angegebene Bedeutung haben,

mit Nitritverbindungen der Formel (IX),

R-O-N=O (IX)

in welcher

R für Wasserstoff, Alkyl oder für ein Alkalimetallkation steht,

in Gegenwart einer Halogenwasserstoffsäure wie beispielsweise Chlorwasserstoffsäure oder Bromwasserstoffsäure oder in Gegenwart eines Haloforms wie beispielsweise Chloroform oder Bromoform bei Temperaturen zwischen -20°C und +80°C in üblicher Art und Weise diazotiert (vgl. z.B. "Organikum" 15.Auflage VEB Deutscher Verlag der Wissenschaften, Berlin 1981 S. 652 ff; J. Chem. Soc. C, 1966, 1249 oder Rev. Latinoam. Quim. 13, 100-102 [1982]).

Die 5-Amino-1-aryl-pyrazole der Formel (VIII) sind teilweise bekannt (vgl. z.B. EP 26 034, EP 53 678 oder EP 34 945, sowie DE-OS 3 226 496, DE-OS 3 408 727 oder DE-OS 3 420 985), teilweise sind sie Gegenstand eigener noch nicht veröffentlichter Patentanmeldungen (vgl. DE-P 3 402 308 vom 24.01.1984; vgl. auch DE-P 35 20 330 vom 07.06.1985 und DE-P 35 20 327 vom 07.06.1985 und die Herstellungsbeispiele).

Man erhält sie beispielsweise, wenn man Aryl-hydrazine der Formel (X),

$Ar-NH-NH_2$ (X)

in welcher

Ar die oben angegebene Bedeutung hat,

(α) mit Acrylnitril-Derivaten der Formel (XI),

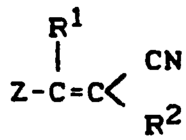 (XI)

in welcher

$R^1$ und $R^2$ die oben angegebene Bedeutung haben und

Z für Halogen, Hydroxy, Alkoxy oder Dialkylamino steht,

oder

(β) mit 2-Halogenacrylnitrilen der Formel (XII),

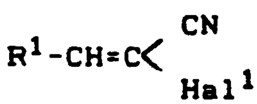 (XII)

in welcher

$R^1$ die oben angegebene Bedeutung hat und

$Hal^1$ für Halogen, insbesondere für Chlor oder Brom steht,

entweder zunächst in einer 1. Stufe, gegebenenfalls in Gegenwart eines Verdünnungsmittels wie beispielsweise Eisessig oder Ethanol, sowie gegebenenfalls in Gegenwart eines Reaktionshilfsmittels wie beispielsweise Natriumacetat bei Temperaturen zwischen -20°C und +20°C umsetzt zu den Arylhydrazin-Derivaten der Formel (XIII),

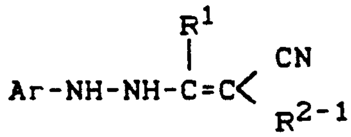

(XIII)

in welcher

Ar und $R^1$ die oben angegebene Bedeutung haben und

$R^{2-1}$ für Halogen, Cyano oder Nitro steht

und diese in einer 2. Stufe, gegebenenfalls in Gegenwart eines Verdünnungsmittels wie beispielsweise Ethylenglykolmonoethylether und gegebenenfalls in Gegenwart eines sauren Katalysators wie beispielsweise Schwefelsäure oder Phosphorsäure bei Temperaturen zwischen +50°C und +150°C cyclisiert,

oder direkt in einem Reaktionsschritt, ohne Isolierung der Zwischenstufe der Formel (XIII) gegebenenfalls in Gegenwart eines Verdünnungsmittels beispielsweise Ethylenglykolmonoethylether oder Ethanol bei Temperaturen zwischen +50°C und +150°C cyclisiert und gegebenenfalls die nach der Variante (β) erhältlichen in 4-Stellung unsubstituierten 5-Aminopyrazole der Formel (XIV),

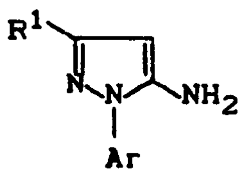

(XIV)

in welcher

$R^1$ und Ar die oben angegebenen Bedeutungen haben,

in einer Folgereaktion mit einem Nitrierungsmittel wie beispielsweise Salpetersäure gegebenenfalls in Gegenwart eines Verdünnungsmittels wie beispielsweise Eisessig und gegebenenfalls in Gegenwart eines Reaktionshilfsmittels wie beispielsweise Acetanhydrid bei Temperaturen zwischen -20°C und +50°C nitriert.

Dabei kann es gegebenenfalls von Vorteil sein, vor der Nitrierungsreaktion die Aminogruppe in der 5-Position des Pyrazolringes mit Hilfe üblicher Schutzgruppentechnik, beispielsweise durch Acylierung, zu schützen und die Aminoschutzgruppe nach erfolgter Nitrierung ebenfalls in üblicher Art und Weise beispielsweise durch Verseifung mit wässriger oder alkoholischer Base wieder abzuspalten.

Die Arylhydrazine der Formel (X) sind bekannt (vgl. z.B. US-PS 4 127 575; US-PS 3 609 158; DE-OS 2 558 399; J. Chem. Soc. C, 1971, 167-174) oder können nach bekannten Verfahren in einfacher analoger Weise erhalten werden (vgl. z.B Houben-Weyl "Methoden der organischen Chemie" Band X/2, S. 203, Thieme Verlag Stuttgart, 1967), indem man beispielsweise die bekannten Aniline bzw. Pyridylamine der Formel (XV),

$$Ar\text{-}NH_2 \qquad (XV)$$

in welcher

Ar die oben angegebene Bedeutung hat,

mit Natriumnitrit in Gegenwart einer Säure wie beispielsweise Schwefelsäure, und dann mit Zinn-II-chlorid ebenfalls in Gegenwart einer Säure wie beispielsweise Salzsäure bei Temperaturen zwischen -20°C und +80°C umsetzt oder indem man Halogenaromate der Formel (XVI),

$$Ar\text{-}Hal^2 \qquad (XVI)$$

in welcher

Ar die oben angegebene Bedeutung hat und

$Hal^2$ für Halogen, insbesondere für Fluor, Chlor oder Brom steht,

mit Hydrazinhydrat gegebenenfalls in Gegenwart eines Verdünnungsmittels wie beispielsweise Pyridin oder Dioxan bei Temperaturen zwischen 0° und 150° C umsetzt.

Die Nitritverbindungen der Formel (IX), die Acrylnitril-Derivate der Formel (XI), die 2-Halogenacrylnitrile der Formel (XII), die Aniline und Pyridylamine der Formel (XV) und die Halogenaromate der Formel (XVI) sind allgemein bekannte Verbindungen der organischen Chemie.

Die zur Durchführung des erfindungsgemäßen Verfahrens (a) weiterhin als Ausgangsstoffe benötigten Hydrazinderivate sind durch die Formel (III) allgemein definiert. In dieser Formel (III) stehen $R^{8-1}$ und $R^{8-2}$unabhängig voneinander vorzugsweise jeweilsfür Wasserstoff oder für jeweils gegebenenfalls einfach oder mehrfach, gleich oder verschieden substituiertes Alkyl, Alkenyl oder Alkinyl mit jeweils bis zu 8 Kohlenstoffatomen, wobei als Substituenten jeweils in Frage kommen: Halogen, Cyano, Nitro, Hydroxy, Carboxy, jeweils geradkettiges oder verzweigtes Alkoxy oder Alkoxycarbonyl mit jeweils 1 bis 8 Kohlenstoffatomen im Alkylteil, oder gegebenenfalls durch Alkyl, Alkoxy, Alkylsulfonyl, Alkenyl oder Alkinyl mit jeweils bis zu 4 Kohlenstoffatomen substituiertes Aminocarbonyl, wobei das Stickstoffatom des Aminocarbonylrestes auch Teil eines gesättigten 3- bis 7-gliedrigen Heterocyclus sein kann, der gegebenenfalls auch 1 oder 2 weitere Heteroatome, insbesondere Stickstoff, Sauerstoff oder Schwefel, enthalten kann; außerdem für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen,

$R^{8-1}$ und $R^{8-2}$ stehen insbesondere unabhängig voneinander jeweils für Wasserstoff, für jeweils gegebenenfalls ein- bis dreifach, gleich oder verschieden substituiertes Methyl, Ethyl, n- oder i-Propyl, n-, i-, s- oder t-Butyl, Allyl, Butenyl, Propargyl oder Butinyl, wobei als Substituenten jeweils in Frage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N-Methyl-N-ethylaminocarbonyl, N-Methyl-N-propylaminocarbonyl, N-Ethyl-N-propylaminocarbonyl, N-Methyl-N-methoxyaminocarbonyl, N,N-Diallylaminocarbonyl, N-Methyl-N-propargylaminocarbonyl, N-Methylsulfonylaminocarbonyl, N-Ethylsulfonylaminocarbonyl, Pyrrolidin-1-ylcarbonyl, Piperidin-1-ylcarbonyl, Morpholin- 4-ylcarbonyl oder Perhydroazepin-1-ylcarbonyl oder für jeweils gegebenenfalls ein- bis fünffach, gleich oder verschieden durch Chlor, Methyl oder Ethyl substituiertes Cyclopropyl, Cyclopentyl, Cyclohexyl oder Cycloheptyl.

Die Hydrazin-Derivate der Formel (III) sind allgemein bekannte Verbindungen der organischen Chemie.

Die zur Durchführung des erfindungsgemäßen Verfahrens (b) als Ausgangsstoffe benötigten 5-Hydrazino-1-arylpyrazole sind durch die Formel (Ia) oder (Ib) allgemein definiert. In dieser Formel (Ia) und (Ib) stehen $R^1$, $R^2$ und Ar vorzugsweise für diejenigen Reste, die bereits

im Zusammenhang mit der Beschreibung der erfindungsgemäßen Stoffe der Formel (I) als bevorzugt für diese Substituenten genannt wurden.

$R^{8-1}$ und $R^{8-2}$ stehen vorzugsweise unabhängig voneinander jeweils für diejenigen Substituenten, die bereits im Zusammenhang mit der Beschreibung der Vorprodukte der Formel (III) für diese Substituenten genannt wurden. Die 5-Hydrazino-1-aryl-pyrazole der Formeln (Ia) und (Ib) sind erfindungsgemäße Verbindungen und erhältlich mit Hilfe des erfindungsgemäßen Verfahrens (a).

Die zur Durchführung des erfindungsgemäßen Verfahrens (b-α) als Ausgangsstoffe benötigten Alkylierungsmittel sind durch die Formel (IV) allgemein definiert. In dieser Formel (IV) steht $R^{8-3}$ vorzugsweise für jeweils gegebenenfalls einfach oder mehrfach, gleich oder verschieden substituiertes Alkyl, Alkenyl oder Alkinyl mit jeweils bis zu 8 Kohlenstoffatomen, wobei als Substituenten jeweils in Frage kommen: Halogen, Cyano, Nitro, Hydroxy, Carboxy, jeweils geradkettiges oder verzweigtes Alkoxy oder Alkoxycarbonyl mit jeweils 1 bis 6 Kohlenstoffatomen im Alkylteil oder gegebenenfalls durch Alkyl, Alkoxy, Alkylsulfonyl, Alkenyl oder Alkinyl mit jeweils bis zu 4 Kohlenstoffatomen substituiertes Aminocarbonyl, wobei das Stickstoffatom des Aminocarbonylrestes auch Teil eines gesättigten 3- bis 7-gliedrigen Heterocyclus sein kann, der gegebenenfalls auch 1 oder 2 weitere Heteroatome, insbesondere Stickstoff, Sauerstoff oder Schwefel, enthalten kann; außerdem für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen.

$R^{8-3}$ steht insbesondere für jeweils gegebenenfalls ein- bis dreifach, gleich oder verschieden substituiertes Methyl, Ethyl, n- oder i-Propyl, n-, i-, s- oder t-Butyl, Allyl, Butenyl, Propargyl oder Butinyl, wobei als Substituenten jeweils in Frage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N-Methyl-N-ethylaminocarbonyl, N-Methyl-N-propylaminocarbonyl, N-Ethyl-N-propylaminocarbonyl, N-Methyl-N-methoxyaminocarbonyl, N,N-Diallylaminocarbonyl, N-Methyl-N-propargylaminocarbonyl, N-Methylsulfonylaminocarbonyl, N-Ethylsulfonylaminocarbonyl, Pyrrolidin-1-ylcarbonyl, Piperidin-1-ylcarbonyl, Morpholin-4- ylcarbonyl oder Perhydroazepin-1-ylcarbonyl oder für jeweils gegebenenfalls ein- bis fünffach, gleich oder verschieden durch Chlor, Methyl und/oder Ethyl substituiertes Cyclopropyl, Cyclopentyl, Cyclohexyl oder Cycloheptyl.

$A^1$ steht vorzugsweise für Chlor, Brom oder Iod, für p-Toluolsulfonyloxy oder Methoxysulfonyloxy.

Die Alkylierungsmittel der Formel (IV) sind allgemein bekannte Verbindungen der organischen Chemie.

Die zur Durchführung des erfindungsgemäßen Verfahrens (b-β) weiterhin als Ausgangsstoffe benötigten Acylierungsmittel sind durch die Formel (V) allgemein definiert. In dieser Formel (V) stehen $R^6$ und X vorzugsweise für diejenigen Reste, die bereits im Zusammenhang mit der Beschreibung der erfindungsgemäßen Stoffe der Formel (I) als bevorzugt für diese Substituenten genannt wurden.

$A^2$ steht vorzugsweise für Chlor oder Brom oder für einen Rest $R^6-\underset{\underset{O}{\|}}{C}-O-$ , wobei

$R^6$ die oben angegebene Bedeutung hat.

Die Acylierungsmittel der Formel (V) sind ebenfalls allgemein bekannte Verbindungen der organischen Chemie.

Die zur Durchführung des erfindungsgemäßen Verfahrens (b-γ)weiterhin als Ausgangsstoffe benötigten Iso(thio)-cyanate sind durch die Formel (VI) allgemein definiert. In dieser Formel (VI) steht X vorzugsweise für diejenigen Reste, die bereits im Zusammenhang mit der Beschreibung der erfindungsgemäßen Stoffe der Formel (I) als bevorzugt für diesen Substituenten genannt wurden.

$R^{6-1}$ steht vorzugsweise für geradkettiges oder verzweigtes Alkyl mit 1 bis 4 Kohlenstoffatomen oder für gegebenenfalls einfach oder mehrfach, gleich oder verschieden substituiertes Phenyl, wobei als Substituenten in Frage kommen: Halogen, jeweils geradkettiges oder verzweigtes

Alkyl, Alkoxy oder Halogenalkyl mit jeweils 1 bis 4 Kohlenstoffatomen und im Fall des Halogenalkyl mit 1 bis 9 gleichen oder verschiedenen Halogenatomen.

$R^{6-1}$ steht insbesondere für Methyl, Ethyl oder für gegebenenfalls ein- bis dreifach, gleich oder verschieden durch Fluor, Chlor, Methyl, Methoxy oder Trifluormethyl substituiertes Phenyl.

Die Iso(thio)cyanate der Formel (VI) sind ebenfalls allgemein bekannte Verbindungen der organischen Chemie.

Die zur Durchführung des erfindungsgemäßen Verfahrens (b-δ) weiterhin als Ausgangsstoffe benötigten Sulfonylierungsmittel sind durch die Formel (VII) allgemein definiert. In dieser Formel (VII) steht $R^7$ vorzugsweise für diejenigen Reste, die bereits im Zusammenhang mit der Beschreibung der erfindungsgemäßen Stoffe der Formel (I) als bevorzugt für diese Substituenten genannt wurden.

$A^3$ steht vorzugsweise für Chlor oder Brom.

Die Sulfonylierungsmittel der Formel (VII) sind ebenfalls allgemein bekannte Verbindungen der organischen Chemie.

Als Verdünnungsmittel zur Durchführung des erfindungsgemäßen Verfahrens (a) kommen inerte organische Lösungsmittel in Frage.

Hierzu gehören insbesondere aliphatische oder aromatische Kohlenwasserstoffe wie beispielsweise Benzin, Benzol, Toluol, Xylol, Petrolether, Hexan, Cyclohexan oder Ether wie Diethylether, Dioxan, Tetrahydrofuran oder Ethylenglykoldimethyl- oder-diethylether.

Die Reaktionstemperaturen können bei der Durchführung des erfindungsgemäßen Verfahrens (a) in einem größeren Bereich variiert werden. Im allgemeinen arbeitet man bei Temperaturen zwischen 20°C und 150°C, vorzugsweise bei Temperaturen zwischen 50°C und 120°C.

Zur Durchführung des erfindungsgemäßen Verfahrens (a) setzt man pro Mol an 1-Aryl-5-halogen-pyrazol der Formel (II) im allgemeinen 1,0 bis 50,0 Mol, vorzugsweise 1,0 bis 20,0 Mol an Hydrazin-Derivat der Formel (III) ein. In Abhängigkeit von Verdünnungsmittel, Reaktionstemperatur, Molverhältnissen, Art der Substituenten $R^{8-1}$ bzw. $R^{8-2}$ und allgemeiner Reaktionsführung erhält man bei Verwendung von substituierten Hydrazin-Derivaten der Formel (III), bei denen $R^{8-1}$ und/oder $R^{8-2}$ verschieden von Wasserstoff sind, entweder α-substituierte Produkte der Formel (Ia) oder β-substituierte Produkte der Formel (Ib) (vgl. z.B Houben-Weyl "Methoden der organischen Chemie" Band X/2, S. 203, Thieme Verlag Stuttgart 1967; vgl. auch Herstellungsbeispiele). Die Reaktionsdurchführung, Aufarbeitung und Isolierung der Reaktionsprodukte (Ia) bzw. (Ib) erfolgen in üblicher Art und Weise.

Als Verdünnungsmittel zur Durchführung der erfindungsgemäßen Verfahren (b-α), (b-β), (b-γ) und (b-δ) kommen ebenfalls inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder-diethylether, Nitrile wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die erfindungsgemäßen Verfahren (b-α), (b-β), (b-γ) und (b-δ) werden gegebenenfalls in Gegenwart eines Säurebindemittels oder eines basischen Reaktionshilfsmittels durchgeführt.

Als solche kommen alle üblichen anorganischen oder organischen Basen in Frage. Hierzu gehören beispielsweise Alkalimetallhydroxide, wie Natriumhydroxdid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen(DBU).

Die Reaktionstemperaturen können bei der Durchführung der erfindungsgemäßen Verfahren (b-α), (b-β), (b-γ) und (b-δ) in einem größeren Bereich variiert werden. Im

allgemeinen arbeitet man bei Temperaturen zwischen -20°C und +120°C, vorzugsweise bei Temperaturen zwischen 0°C und +100°C.

Zur Durchführung der erfindungsgemäßen Verfahren (b-α), (b-β), (b-γ) und (b-δ) setzt man pro Mol an 5-Hydrazino-1-aryl-pyrazol der Formel (Ia) bzw. (Ib) im allgemeinen 1,0 bis 20,0 Mol, vorzugsweise 1,0 bis 15,0 Mol an Alkylierungsmittel der Formel (IV) bzw. an Acylierungsmittel der Formel (V) bzw. an Iso(thio)cyanat der Formel (VI) oder an Sulfonylierungsmittel der Formel (VII) und gegebenenfalls 1,0 bis 3,0 Mol, vorzugsweise 1,0 bis 2,0 Mol an Säurebindemittel oder basischem Reaktionshilfsmittel ein. Die Reaktionsdurchführung, Aufarbeitung und Isolierung der Reaktionsprodukte der Formel (I) erfolgen in allgemein üblicher Art und Weise.

Auch hierbei kommt es in Abhängigkeit von Verdünnungsmittel, Reaktionstemperatur, Base, Molverhältnissen, Art der Abgangsgruppen $A^1$, $A^2$ und $A^3$, Art der beteiligten Substituenten $R^6$, $R^7$, $R^{8-1}$, $R^{8-2}$ und $R^{8-3}$ und allgemeinen Reaktionsbedingungen zu unterschiedlichen Substitutionsstellen an der Hydrazingruppierung (α- oder β-Position) und zu unterschiedlichen Substitutionsgraden (mono- oder di-Substitution) (vgl. z.B. Synthesis 1983, 157-158). Mono-substituierte Produkte der Formel (I) kann man gegebenenfalls in einer wiederholten Reaktion mit veränderten Reaktionsbedingungen wiedereinsetzen, um auf diese Weise zu gemischt-substituierten Produkten zu kommen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in mono- und dikotylen Kulturen wie beispielsweise Soja, Baumwolle oder Weizen einsetzen.

In entsprechenden Aufwandmengen zeigen die erfindungsgemäßen Wirkstoffe darüberhinaus auch eine blattinsektizide Wirksamkeit.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4-(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-trifluormethylphenyl)-harnstoff; N,N-Dimethyl-N'-(3-chlor-4-methylphenyl)-harnstoff; N,N-Dimethyl-N'-(4-isopropylphenyl)-harnstoff; 4-Amino-6-t-butyl-3-ethylthio-1,2,4-triazin-5-(4H)-on; 2,4-Dichlorphenoxyessigsäure; 2,4-Dichlorphenoxy-propionsäure; (2-Methyl-4-chlorphenoxy)-essigsäure; (4-Chlor-2-methylphenoxy)-propionsäure; Chloressigsäure-N-(methoxymethyl)-2,6-diethylanilid; 2-Ethyl-6-methyl-N-(1-methyl-2-methoxyethyl)-chloracetanilid; 2,6-Dinitro-4-trifluormethyl-N,N-dipropylanilin; 2-[4-(3,5-Dichlorpyrid-2-yloxy)-phenoxy]-propionsäure-2-(benzyloxyethylester), -(trimethylsilylmethylester) oder -(2,2-diethoxyethylester); 2-[1-(Ethoxyamino)-butyliden]-5-(2-ethylthiopropyl)-1,3-cylcohexandion; Methyl-5-(2,4-dichlorphenoxy)-2-nitrobenzoat; 3,5-Diiod-4-hydroxybenzonitril; 3-Isopropyl-2,1,3-benzothiadiazin-4-on-2,2-dioxid; 2-Chlor-N-{[(4-methoxy-6-methyl-1,3,5-triazin-2-yl)-amino]-carbonyl}-benzolsulfonamid; 4-Ethylamino-2-t-butylamino-6-methylthio-s-triazin; 2-{4-[(3-Chlor-5-(trifluormethyl)-2-pyridyl)-oxy]-phenoxy}-propansäure bzw.-propansäureethylester; 5-(2-Chlor-4-trifluormethyl-phenoxy)-2-nitro-benzoesäure; N-(1-Ethylpropyl)-3,4-dimethyl-2,6-dinitroanilin; N-Methyl-2-(1,3-benzthiazol-2-yloxy)-acetanilid; N,N-Diisopropyl-S-(2,3,3-trichlorallyl)-thiolcarbamat; sowie 2-[5-Methyl-(1-methylethyl)-4-oxo-2-imidazolin-2-yl]-3-chinolincarbonsäure sind gegebenenfalls von Vorteil. Einige Mischungen zeigen überraschenderweise auch synergistische Wirkung.

Auch eine Mischung mit anderen bekannten Wirkstoffen wie Fungiziden, Insektiziden, Akariziden, Nematiziden, Schutzstoffen gegen Vogelfraß, Pflanzennährstoffen und Bodenstrukturverbesserern ist möglich.

Die Wirkstoffe können als solche, in Form ihrer Formulierungen oder den daraus durch weiteres Verdünnen bereiteten Anwendungsformen wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

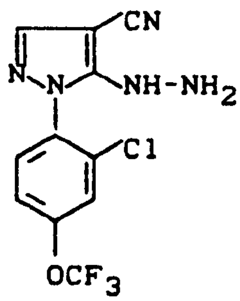

18,3 g (0,05 Mol) 5-Brom-4-cyano-1-(2-chlor-4-trifluormethoxy-phenyl)-pyrazol und 50 ml (1,0 Mol) Hydrazinhydrat werden in 300 ml Dioxan 18 Stunden unter Rückfluß erhitzt. Die erkaltete Reaktionsmischung wird im Vakuum eingeengt, der Rückstand in Chloroform gelöst, nacheinander mit Wasser, verdünnter wässriger Salzsäure, wässriger Natriumhydrogencarbonatlösung und wieder mit Wasser gewaschen, über Natriumsulfat getrocknet und im Vakuum vom Lösungsmittel befreit.

Man erhält 9,5 g (60 % der Theorie) an 4-Cyano-5-hydrazino-1-(2-chlor-4-trifluormethoxy-phenyl)-pyrazol vom Schmelzpunkt 163°C (Zers.).

Herstellung der Ausgangsverbindung:

Beispiel (II-1):

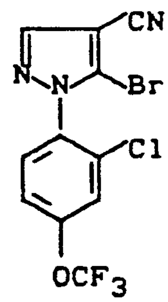

(II-1)

Zu 9,1 g (0,03 Mol) 5-Amino-4-cyano-1-(2-chlor-4-trifluormethoxy-phenyl)-pyrazol (vgl. DE-OS 3 420 985) in 100 ml Bromwasserstoffsäure gibt man bei 0°C bis 5°C unter Rühren eine Lösung von 3,6 g (0,052 Mol) Natriumnitrit in 9 ml Wasser. Nach beendeter Zugabe läßt man die Temperatur auf 20°C steigen und rührt weitere 6 Stunden bei dieser Temperatur; das kristalline Produkt wird abgesaugt, in Wasser aufgenommen, mit Natriumhydrogencarbonatlösung neutralisiert, abgesaugt und getrocknet.

Man erhält 10 g (91 % der Theorie) an 5-Brom-4-cyano-1-(2-chlor-4-trifluormethoxy-phenyl)-pyrazol vom Schmelzpunkt 83°C.

Beispiel 2:

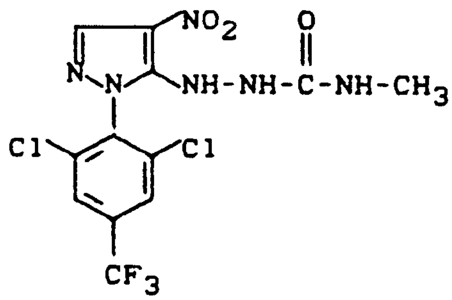

11g (0,03 Mol) an 5-Hydrazino-4-nitro-1-(2,6-dichlor-4-trifluormethyl-phenyl)-pyrazol und 2 g (0,035 Mol) Methylisocyanat in 50 ml Essigsäureethylester werden mit 2 Tropfen Triethylamin versetzt, 12 Stunden bei 20°C gerührt, auf 5°C abgekühlt und der ausgefallene Niederschlag abgesaugt.

Man erhält 5 g (40 % der Theorie) an 5-[(β-Methylaminocarbonyl)-hydrazino]-4-nitro-1-(2,6-dichlor-4-trifluormethyl-phenyl)-pyrazol vom Schmelzpunkt 207°C -210°C.

Beispiel 3:

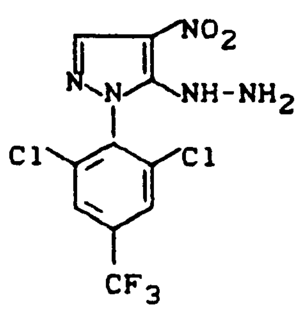

11 g (0,03 Mol) 5-Chlor-4-nitro-1-(2,6-dichlor-4-trifluormethyl-phenyl)-pyrazol und 6 ml (0,12 Mol) Hydrazinhydrat in 100 ml Dioxan werden 16 Stunden bei 20°C stehengelassen. Zur Aufarbeitung gießt man die Reaktionsmischung in Wasser, extrahiert mehrfach mit Chloroform, trocknet die vereinigten organischen Phasen über Natriumsulfat und entfernt das Lösungsmittel im Vakuum. Der Rückstand kristallisiert bei Anreiben mit Ligroin.

Man erhält 8 g (75 % der Theorie) an 5-Hydrazino-4-nitro-1-(2,6-dichlor-4-trifluormethyl-phenyl)-pyrazol vom Schmelzpunkt 133°C-135°C.

Herstellung der Ausgangsverbindungen:

Beispiel (II-2)

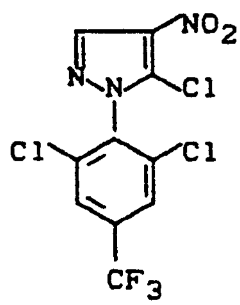

(II-2)

62 g (0,2 Mol) 5-Chlor-1-(2,6-dichlor-4-trifluormethylphenyl)-pyrazol werden in einer Mischung aus 260 ml konzentrierter Schwefelsäure und 70 ml Wasser suspendiert und bei 60°C tropfenweise mit einem Gemisch aus 40 ml

konzentrierter Schwefelsäure und 40 ml konzentrierter Salpetersäure versetzt. Nach beendeter Zugabe wird noch 12 Stunden bei 60° C gerührt, abgekühlt und auf Eis gegossen. Das sich ölig abscheidende Reaktionsprodukt wird mit Toluol aufgenommen, die organische Phase mehrmals mit Wasser und dann mit Natriumhydrogencarbonat-Lösung gewaschen und anschließend über Natriumsulfat getrocknet. Nach Abdestillieren des Lösungsmittels im Vakuum wird der Rückstand aus wenig Petrolether umkristallisiert.

Man erhält 32 g (44 % der Theorie) an 5-Chlor-1-(2,6-dichlor-4-trifluormethylphenyl)-4-nitro-pyrazol vom Schmelzpunkt 80° C-84° C.

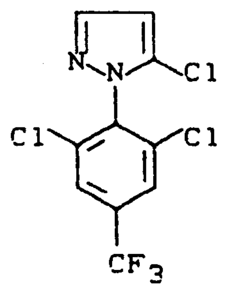

Ein Gemisch aus 60 g (0,2 Mol) 1-(2,6-Dichlor-4-trifluormethyl-phenyl)-5-hydroxy-pyrazol und 250 ml Phosphoroxychlorid wird im Autoklaven 20 Stunden auf 160° C erhitzt. Anschließend wird der abgekühlte Reaktionsansatz auf Eis gegossen und das überschüssige Phosphoroxychlorid vorsichtig unter Kühlen mit Natronlauge hydrolysiert. Der Niederschlag wird abgesaugt, mehrmals mit Wasser gewaschen und getrocknet.

Man erhält 32 g (51 % der Theorie) an 5-Chlor-1-(2,6-dichlor-4-trifluormethyl-phenyl)-pyrazol vom Schmelzpunkt 70°C-72°C.

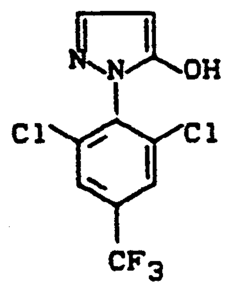

In eine Lösung aus 30 g (0,75 Mol) Natriumhydroxid in 1000 ml Wasser trägt man bei 80-85°C portionsweise unter Rühren 105 g (0,253 Mol) fein gepulverten β-(2,6-Dichlor-4-trifluormethyl-phenyl)-hydrazinomethylenmalonsäurediethylester und rührt anschließend weitere 48 Stunden bei 97-98°C. Die erkaltete Reaktionsmischung wird mit konzentrierter Salzsäure vorsichtig angesäuert auf pH 2 und der so erhaltene Niederschlag abgesaugt und auf Ton getrocknet.

Man erhält 100 g (67 % der Theorie) an 5-Hydroxy-1-(2,6-dichlor-4-trifluormethyl-phenyl)-pyrazol vom Schmelzpunkt 223°C-225°C.

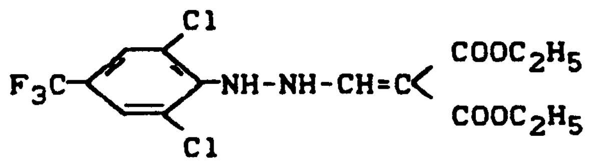

Zu einer Lösung von 122,5 g (0,5 Mol) 2,6-Dichlor-4-trifluormethyl-phenylhydrazin in 1000 ml Ethanol tropft man bei 70°C-75°C innerhalb von 30 Minuten unter Rühren 115 g (0,53 Mol) Ethoxymethylenmalonsäurediethylester und rührt nach beendeter Zugabe weitere 5 Stunden bei 70°C bis 75°C. Zur Aufarbeitung entfernt man das Lösungsmittel im Vakuum, reibt den Rückstand mit Wasser an, saugt ab und trocknet auf Ton.

Man erhält 202 g (97 % der Theorie) an β-(2,6-Dichlor-4-trifluormethylphenyl)-hydrazinomethylen-malonsäurediethylester vom Schmelzpunkt 73°C-83°C.

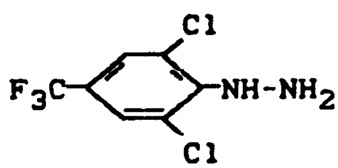

6,2 g (0,025 Mol) 3,4,5-Trichlor-trifluormethylbenzol und 6,25 g (0,125 Mol) Hydrazinhydrat werden in 12 ml Pyridin 48 Stunden bei 115-120°C unter Rückfluß erhitzt. Zur Aufarbeitung destilliert man das Lösungsmittel ab, nimmt den Rückstand in Wasser auf und extrahiert dreimal mit jeweils ca. 30 ml Dichlormethan. Die vereinigten

organischen Phasen werden über Magnesiumsulfat getrocknet, im Vakuum eingeengt und anschließend destilliert.

Man erhält 5,1 g (83 % der Theorie) an 2,6-Dichlor-4-trifluormethylphenylhydrazin vom Schmelzpunkt 56 bis 57°C.

In entsprechender Weise und gemäß den allgemeinen Angaben zur Herstellung erhält man die folgenden 1-Aryl-5-hydrazino-pyrazole der Formel (I):

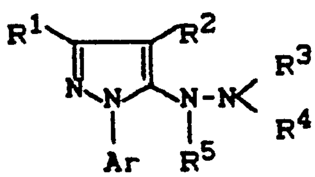

(I)

**Tabelle 2:**

| Beispiel Nr. | $R^1$ | $R^2$ | $R^3$ | $R^4$ | $R^5$ | Ar | Schmelzpunkt/°C |
|---|---|---|---|---|---|---|---|
| 4 | H | CN | $C_2H_5-\overset{O}{\overset{\Vert}{C}}-$ | H | H | 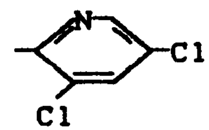 | 173 |
| 5 | H | $NO_2$ | H | H | H | 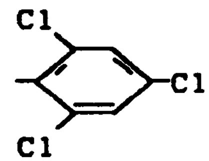 | 165-170 (Zers.) |
| 6 | H | CN | H | H | H | 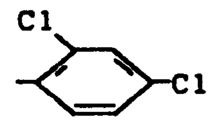 | 199-203 |
| 7 | H | CN | H | H | H | 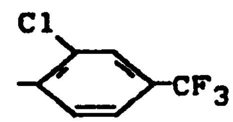 | 141-145 |
| 8 | H | $NO_2$ | H | H | H | 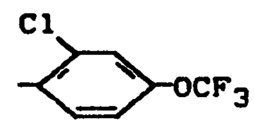 | 169 |

**Tabelle 2 (Fortsetzung):**

| Beispiel Nr. | $R^1$ | $R^2$ | $R^3$ | $R^4$ | $R^5$ | Ar | Schmelzpunkt/°C |
|---|---|---|---|---|---|---|---|
| 9 | H | $NO_2$ | 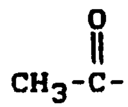 | H | H | 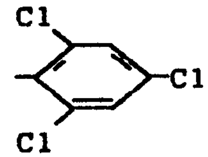 | 206-208 (Zers.) |
| 10 | H | CN | H | H | H | 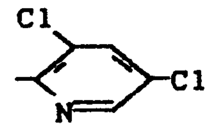 | 216 |
| 11 | H | CN | 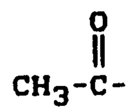 | H | H | 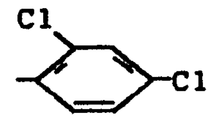 | 90 (Zers.) |
| 12 | H | $NO_2$ | 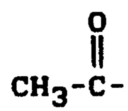 | H | H | 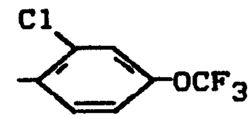 | 168 |
| 13 | H | CN | 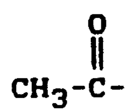 | H | H | 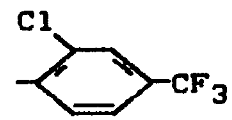 | 208 |

**Tabelle 2 (Fortsetzung):**

| Beispiel Nr. | $R^1$ | $R^2$ | $R^3$ | $R^4$ | $R^5$ | Ar | Schmelzpunkt/°C |
|---|---|---|---|---|---|---|---|
| 14 | H | CN | 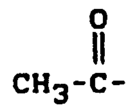 | H | H | 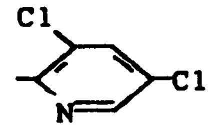 | 179-180 |
| 15 | H | CN | 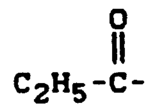 | H | H | 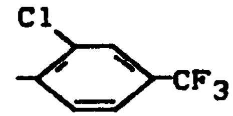 | 203-205 |
| 16 | H | CN | 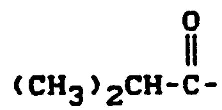 | H | H | 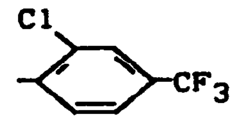 | 187-189 |
| 17 | $CH_3$ | $NO_2$ | H | H | H | 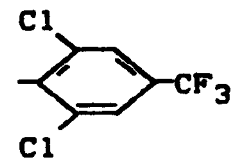 | 178-182 |
| 18 | H | $NO_2$ | 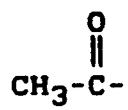 | H | H | 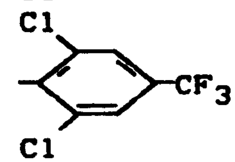 | 205-207 |

Tabelle 2 (Fortsetzung):

| Beispiel Nr. | $R^1$ | $R^2$ | $R^3$ | $R^4$ | $R^5$ | Ar | Schmelzpunkt/°C |
|---|---|---|---|---|---|---|---|
| 19 | H | $NO_2$ | 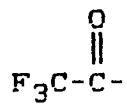 | H | H | 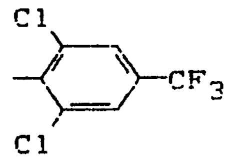 | 88-92 |
| 20 | H | $NO_2$ | 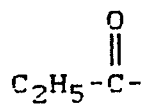 | H | H | 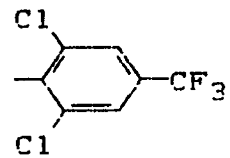 | 160-162 |
| 21 | H | CN | H | H | $CH_3$ | 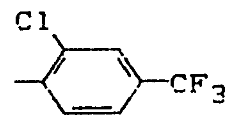 | Öl ($^1$H-NMR: δ3,25 (s,3H) ($CDCl_3$/TMS) |
| 22 | H | $NO_2$ | H | H | H | 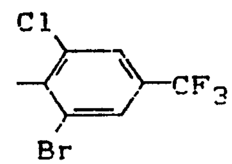 | 80-86 |

Tabelle 2 (Fortsetzung):

| Beispiel Nr. | $R^1$ | $R^2$ | $R^3$ | $R^4$ | $R^5$ | Ar | Schmelzpunkt/°C |
|---|---|---|---|---|---|---|---|
| 23 | H | $NO_2$ | 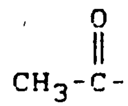 | H | H | 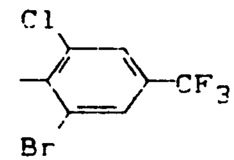 | 75-105 |
| 24 | H | $NO_2$ | 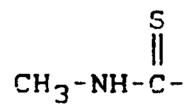 | H | H | 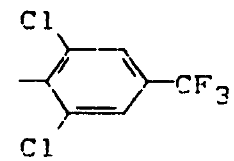 | 182-184 |
| 25 | H | $NO_2$ | $-SO_2-CH_3$ | H | H | 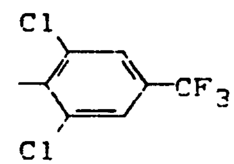 | 100-120 |
| 26 | H | $NO_2$ | $-CO-OCH_3$ | H | H | 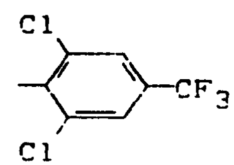 | 203-208 |

Anwendungsbeispiele:

In den folgenden Anwendungsbeispielen wurde die nachstehend aufgeführte Verbindung als Vergleichsubstanz eingesetzt:

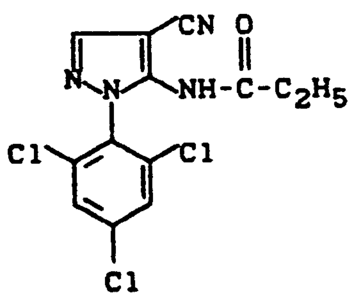

(A)

4-Cyano-5-propionamido-1-(2,4,6-trichlorphenyl)-pyrazol (bekannt aus DE-OS 3 226 513)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß der Herstellungsbeispiele: 3 und 18.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Nutzpflanzenselektivität gegenüber dem Stand der Technik zeigt in diesem Test z.B. die Verbindung gemäß dem Herstellungsbeispiel: 18.

## Patentansprüche:

1. 1-Aryl-5-hydrazino-pyrazole der Formel (I),

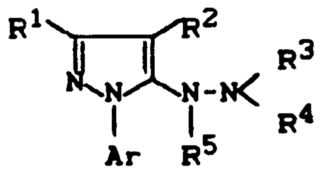

(I)

in welcher

$R^1$ für Wasserstoff oder Alkyl steht,

$R^2$ für Cyano oder Nitro steht,

$R^3$ für Wasserstoff, für jeweils gegebenenfalls substituiertes Alkyl, Alkenyl, Alkinyl oder Cycloalkyl, für einen Rest $-\underset{X}{\underset{\|}{C}}-R^6$ oder für einen Rest $-SO_2-R^7$ steht,

$R^4$ für Wasserstoff, für jeweils gegebenenfalls substituiertes Alkyl, Alkenyl, Alkinyl oder Cycloalkyl, für einen Rest $-\underset{X}{\underset{\|}{C}}-R^6$ oder für einen Rest-$SO_2-R^7$ steht,

$R^5$ für Wasserstoff steht; oder für den Fall, daß $R^4$ für Wasserstoff steht, auch für einen Rest $-\underset{X}{\underset{\|}{C}}-R^6$, für einen Rest-$SO_2-R^7$

oder für jeweils gegebenenfalls substituiertes Alkyl, Alkenyl, Alkinyl oder Cycloalkyl steht,

wobei jeweils

X für Sauerstoff oder Schwefel steht,

$R^6$ für Wasserstoff, Alkyl, Alkoxy, Alkoxyalkyl, Alkylthio, Alkylthioalkyl, Alkylamino, Dialkylamino, Halogenalkyl, Alkenyl, Alkinyl, für gegebenenfalls substituiertes Cycloalkyl oder für jeweils gegebenenfalls substituiertes Aryl, Aryloxy, Arylthio oder Arylamino steht und

$R^7$ für Alkyl, Hydroxyalkyl, Alkoxyalkyl, Halogenalkyl, Alkenyl, Alkinyl, Cycloalkyl oder für jeweils gegebenenfalls substituiertes Aralkyl oder Aryl steht und

Ar für substituiertes Phenyl oder für gegebenenfalls substituiertes Pyridyl steht.

2. 1-Aryl-5-hydrazino-pyrazole der Formel (I) gemäß Anspruch 1, in welcher

$R^1$ für Wasserstoff oder für geradkettiges oder verzweigtes Alkyl mit 1 bis 4 Kohlenstoffatomen steht,

$R^2$ für Cyano oder Nitro steht,

$R^3$ für Wasserstoff, für jeweils gegebenenfalls einfach oder mehrfach, gleich oder verschieden substituiertes Alkyl, Alkenyl oder Alkinyl mit jeweils bis zu 8 Kohlenstoffatomen steht, wobei als Substituenten jeweils in Frage kommen: Halogen, Cyano, Nitro, Hydroxy, Carboxy, jeweils geradkettiges oder verzweigtes Alkoxy oder Alkoxycarbonyl mit jeweils 1 bis 6 Kohlenstoffatomen im Alkylteil, oder gegebenenfalls durch Alkyl, Alkoxy, Alkylsulfonyl, Alkenyl oder Alkinyl mit jeweils bis zu 4 Kohlenstoffatomen substituiertes Aminocarbonyl, wobei das Stickstoffatom des Aminocarbonylrestes auch Teil eines gesättigten 3- bis 7-gliedrigen Heterocyclus sein kann, der gegebenenfalls auch 1 oder 2 weitere Heteroatome, insbesondere Stickstoff, Sauerstoff oder Schwefel, enthalten kann;
außerdem für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen steht,
für einen Rest $-\underset{\underset{X}{\|}}{C}-R^6$

oder für einen Rest-$SO_2$-$R^7$ steht,

$R^4$ für Wasserstoff, für jeweils gegebenenfalls einfach oder mehrfach, gleich oder verschieden substituiertes Alkyl, Alkenyl oder Alkinyl mit jeweils bis zu 8 Kohlenstoffatomen steht, wobei als Substituenten jeweils in Frage kommen: Halogen, Cyano, Nitro, Hydroxy, Carboxy, jeweils geradkettiges oder verzweigtes Alkoxy oder Alkoxycarbonyl mit jeweils 1 bis 6 Kohlenstoffatomen im Alkylteil, oder gegebenenfalls durch Alkyl, Alkoxy, Alkylsulfonyl, Alkenyl oder Alkinyl mit jeweils bis zu 4 Kohlenstoffatomen substituiertes Aminocarbonyl, wobei das Stickstoffatom des Aminocarbonylrestes auch Teil eines gesättigten 3- bis 7-gliedrigen Heterocyclus sein kann, der gegebenenfalls auch 1 oder 2 weitere Heteroatome, insbesondere Stickstoff, Sauerstoff oder Schwefel, enthalten kann;
außerdem für gegebenenfalls einfach oder mehrfach, gleich oder verschieden durch Halogen und geradkettiges oder verzweigtes Alkyl mit 1 bis 4 Kohlenstoffatomen substituiertes Cycloalkyl mit 3 bis 7 Kohlenstoffatomen steht,
für einen Rest $-\underset{\underset{X}{\|}}{C}-R^6$

oder für einen Rest $-SO_2-R^7$ steht,

$R^5$ für Wasserstoff steht; oder für den Fall, daß $R^4$ für Wasserstoff steht, auch für einen Rest $-\underset{\underset{X}{\|}}{C}-R^6$, für einen Rest-$SO_2$-$R^7$ steht, oder für jeweils gegebenenfalls einfach oder mehrfach, gleich oder verschieden substituiertes Alkyl, Alkenyl oder Alkinyl mit jeweils bis zu 8 Kohlenstoffatomen steht, wobei als Substituenten jeweils in Frage kommen: Halogen, Cyano, Nitro, Hydroxy, Carboxy, jeweils geradkettiges oder verzweigtes Alkoxy oder Alkoxycarbonyl mit jeweils bis zu 6 Kohlenstoffatomen im Alkylteil, oder gegebenenfalls durch Alkyl, Alkoxy, Alkylsulfonyl, Alkenyl oder Alkinyl mit jeweils bis zu 4 Kohlenstoffatomen substituiertes Aminocarbonyl, wobei das Stickstoffatom des Aminocarbonylrestes auch Teil eines gesättigten 3- bis 7-gliedrigen Heterocyclus sein kann, der gegebenenfalls 1 oder 2 weitere Heteroatome, insbesondere Stickstoff, Sauerstoff oder Schwefel, enthalten kann; und außerdem für gegebenenfalls einfach oder mehrfach, gleich oder verschieden durch Halogen und geradkettiges oder verzweigtes Alkyl mit 1 bis 4 Kohlenstoffatomen substituiertes Cycloalkyl mit 3 bis 7 Kohlenstoffatomen steht,

wobei jeweils

X für Sauerstoff oder Schwefel steht,

$R^6$ für Wasserstoff, für jeweils geradkettiges oder verzweigtes Alkyl, Alkoxy, Alkoxyalkyl, Alkylthio, Alkylthioalkyl, Alkylamino oder Dialkylamino mit jeweils 1 bis 4 Kohlenstoffatomen in den einzelnen Alkylteilen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkenyl oder Alkinyl mit 2 bis 4 Kohlenstoffatomen, für gegebenenfalls einfach oder mehrfach, gleich oder verschieden durch Halogen, jeweils geradkettiges oder verzweigtes Alkyl mit 1 bis 4 Kohlenstoffatomen oder Halogenalkyl mit 1 bis 4 Kohlenstoffatomen und 1 bis 9 gleichen oder verschiedenen Halogenatomen substituiertes Cycloalkyl mit 3 bis 7 Kohlenstoffatomen oder für jeweils gegebenenfalls einfach oder mehrfach, gleich oder verschieden substituiertes Phenyl, Phenoxy, Phenylthio oder Phenylamino steht, wobei als Phenylsubstituenten ausgewählt sind: Halogen, jeweils geradkettiges oder verzweigtes Alkyl oder Alkoxy mit jeweils 1 bis 4 Kohlenstoffatomen oder geradkettiges oder verzweigtes Halogenalkyl mit jeweils 1 bis 4 Kohlenstoffatomen und 1 bis 9 gleichen oder verschiedenen Halogenatomen, und

$R^7$ für jeweils geradkettiges oder verzweigtes Alkyl, Hydroxyalkyl oder Alkoxyalkyl mit jeweils 1 bis 4 Kohlenstoffatomen in den einzelnen Alkylteilen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkenyl oder Alkinyl mit jeweils 2 bis 4 Kohlenstoffatomen, für Cycloalkyl mit 3 bis 7 Kohlenstoffatomen oder für jeweils gegebenenfalls einfach oder mehrfach, gleich oder verschieden durch Halogen, Cyano, Nitro jeweils geradkettiges oder verzweigtes Alkyl, Alkoxy oder Alkylthio mit jeweils 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen substituiertes Benzyl oder Phenyl steht und

Ar für einfach oder mehrfach, gleich oder verschieden substituiertes Phenyl oder für jeweils gegebenenfalls einfach oder mehrfach, gleich oder verschieden substituiertes 2-Pyridyl, 3-Pyridyl oder 4-Pyridyl steht, wobei als Substituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen im Alkylteil, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^9$, wobei

$R^9$ für Amino, für jeweils geradkettiges oder verzweigtes Alkyl, Alkylamino oder Dialkylamino mit jeweils 1 bis 4 Kohlenstoffatomen in den

einzelnen Alkylteilen oder für geradkettiges oder verzweigtes Halogenalkyl mit 1 bis 4 Kohlenstoffatomen und 1 bis 9 gleichen oder verschiedenen Halogenatomen steht und

m für eine Zahl 0, 1 oder 2 steht.

3. 1-Aryl-5-hydrazino-pyrazole der Formel (I) gemäß Anspruch 1, in welcher

$R^1$ für Wasserstoff, Methyl, Ethyl, n- oder i-Propyl, n-, i-, s- oder t-Butyl steht,

$R^2$ für Cyano oder Nitro steht,

$R^3$ für Wasserstoff, für jeweils gegebenenfalls ein- bis dreifach, gleich oder verschieden substituiertes Methyl, Ethyl, n- oder i-Propyl, n-, i-, s- oder t- Butyl, Allyl, Butenyl, Propargyl oder Butinyl steht, wobei als Substituenten jeweils in Frage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N-Methyl-N-ethylaminocarbonyl, N-Methyl-N-propylaminocarbonyl, N-Ethyl-N-propylaminocarbonyl, N-Methyl-N-methoxyaminocarbonyl, N,N-Diallylaminocarbonyl, N-Methyl-N-propargylaminocarbonyl, N-Methylsulfonylaminocarbonyl, N-Ethylsulfonylaminocarbonyl, Pyrrolidin-1-ylcarbonyl, Piperidin-1-ylcarbonyl, Morpholin-4-ylcarbonyl oder Perhydroazepin-1-ylcarbonyl;
außerdem für jeweils gegebenenfalls ein- bis fünffach, gleich oder verschieden durch Chlor, Methyl oder Ethyl substituiertes Cyclopropyl, Cyclopentyl, Cyclohexyl oder Cycloheptyl steht oder für einen Rest $-\underset{\underset{X}{\|}}{C}-R^6$ oder für einen Rest $-SO_2-R^7$ steht,

$R^4$ für Wasserstoff, für jeweils gegebenenfalls ein- bis dreifach, gleich oder verschieden substituiertes Methyl, Ethyl, n- oder i-Propyl, n-, i-, s- oder t- Butyl, Allyl, Butenyl, Propargyl oder Butinyl steht, wobei als Substituenten jeweils in Frage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N-Methyl-N-ethylaminocarbonyl, N-Methyl-N-propylaminocarbonyl, N-Ethyl-N-propylaminocarbonyl, N-Methyl-N-methoxyaminocarbonyl, N,N-Diallylaminocarbonyl, N-Methyl-N-propargylaminocarbonyl, N-Methylsulfonylaminocarbonyl, N-Ethylsulfonylaminocarbonyl, Pyrrolidin-1-ylcarbonyl, Piperidin-1-ylcarbonyl, Morpholin-4-ylcarbonyl oder Perhydroazepin-1-ylcarbonyl; außerdem für jeweils gegebenenfalls ein- bis fünffach, gleich oder verschieden durch Chlor, Methyl oder Ethyl substituiertes Cyclopropyl, Cyclopentyl, Cyclohexyl oder Cycloheptyl steht und darüberhinaus für einen Rest $-\underset{\underset{X}{\|}}{C}-R^6$ oder für einen Rest $-SO_2-R^7$ steht,

$R^5$ für Wasserstoff steht, oder für den Fall, daß $R^4$ für Wasserstoff steht, auch für einen Rest $-\overset{\overset{X}{\|}}{C}-R^6$, einen Rest $-SO_2-R^7$ oder für jeweils gegebenenfalls ein- bis dreifach, gleich oder verschieden substituiertes Methyl, Ethyl, n- oder i-Propyl, n-, i-, s- oder t- Butyl, Allyl, Butenyl, Propargyl oder Butinyl steht, wobei als Substituenten jeweils in Frage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbon-

0212353

yl, N-Ethylaminocarbonyl, N,N-Dimethylaminocarbonyl, N,N-Diethylaminocarbonyl, N-Methyl-N-ethylaminocarbonyl, N-Methyl- N-propylaminocarbonyl, N-Ethyl-N-propylaminocarbonyl, N-Methyl- N-methoxyaminocarbonyl, N,N-Diallylaminocarbonyl, N-Methyl-N-propargylaminocarbonyl, N-Methylsulfonylaminocarbonyl, N-Ethylsulfonylaminocarbonyl, Pyrrolidin-1-ylcarbonyl, Piperidin-1-ylcarbonyl, Morpholin-4- ylcarbonyl oder Perhydroazepin-1-ylcarbonyl; außerdem für jeweils gegebenenfalls ein- bis fünffach, gleich oder verschieden durch Chlor, Methyl oder Ethyl substituiertes Cyclopropyl, Cyclopentyl, Cyclohexyl oder Cycloheptyl steht,

wobei jeweils

X für Sauerstoff oder Schwefel steht,

$R^6$ für Wasserstoff, Methyl, Ethyl, n- oder i- Propyl, Methoxy, Ethoxy, Methoxymethyl, Ethoxymethyl, Methoxyethyl, Ethoxyethyl, Methylthio, Ethylthio, Methylthiomethyl, Methylamino, Ethylamino, Dimethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, Heptafluor-n-propyl, Allyl, Propargyl, Butenyl, für jeweils gegebenenfalls ein- bis vierfach, gleich oder verschieden durch Fluor, Chlor, Brom, Methyl und Trifluormethyl substituiertes Cyclopropyl,

Cyclopentyl oder Cyclohexyl oder für jeweils gegebenenfalls ein- bis dreifach, gleich oder verschieden durch Chlor, Methyl, Methoxy oder Trifluormethyl substituiertes Phenyl, Phenoxy, Phenylthio oder Phenylamino steht, und

$R^7$ für Methyl, Ethyl, n- oder i-Propyl, n-, i-, s- oder t-Butyl, Hydroxymethyl, Hydroxyethyl, Methoxymethyl, Methoxyethyl, Chlormethyl, Dichlormethyl, Trifluormethyl, Allyl, Butenyl, Cyclopropyl, Cyclopentyl, Cyclohexyl oder für jeweils gegebenenfalls ein- bis dreifach, gleich oder verschieden substituiertes Benzyl oder Phenyl steht, wobei als Substituenten jeweils in Frage kommen: Fluor, Chlor, Brom, Cyano, Nitro, Methyl, Ethyl, Methoxy, Methylthio oder Trifluormethyl,

und

Ar für ein- bis fünffach, gleich oder verschieden substituiertes Phenyl oder für jeweils gegebenenfalls ein- bis vierfach, gleich oder verschieden substituiertes 2-Pyridyl, 3-Pyridyl oder 4-Pyridyl steht, wobei als Substituenten jeweils in Frage kommen: Cyano, Nitro, Fluor, Chlor, Brom, Iod, Methyl, Ethyl, n- oder i-Propyl, n-, i-, s- oder t-Butyl, Methoxy, Ethoxy, Methoxycarbonyl, Ethoxycarbonyl, Trifluormethyl, Trichlormethyl, Difluorchlormethyl, Difluorchlorethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^9$, wobei

$R^9$ für Amino, Methyl, Ethyl, Methylamino, Ethylamino, Dimethylamino, Diethylamino, Fluordichlormethyl, Difluormethyl, Tetrafluorethyl, Trifluorchlorethyl oder Trifluormethyl steht und

m für eine Zahl 0, 1 oder 2 steht.

4. Verfahren zur Herstellung von 1-Aryl-5-hydrazinopyrazolen der Formel (I),

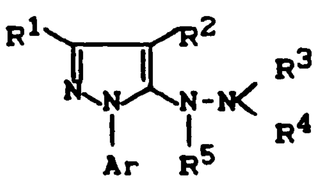

(I)

in welcher

$R^1$ für Wasserstoff oder Alkyl steht,

$R^2$ für Cyano oder Nitro steht,

$R^3$ für Wasserstoff, für jeweils gegebenenfalls substituiertes Alkyl, Alkenyl, Alkinyl oder Cycloalkyl, für einen Rest $-\underset{\underset{X}{\|}}{C}-R^6$

oder für einen Rest $-SO_2-R^7$ steht,

$R^4$ für Wasserstoff, für jeweils gegebenenfalls substituiertes Alkyl, Alkenyl, Alkinyl oder Cycloalkyl, für einen Rest$-\underset{\underset{X}{\|}}{C}-R^6$

oder für einen Rest$-SO_2-R^7$ steht,

$R^5$ für Wasserstoff steht;
oder für den Fall, daß $R^4$ für Wasserstoff steht auch für einen Rest $-\overset{\overset{X}{\|}}{C}-R^6$, für einen Rest$-SO_2-R^7$ oder für jeweils gegebenenfalls substituiertes Alkyl, Alkenyl, Alkinyl oder Cycloalkyl steht,

0212353

wobei jeweils

X für Sauerstoff oder Schwefel steht,

$R^6$ für Wasserstoff, Alkyl, Alkoxy, Alkoxyalkyl, Alkylthio, Alkylthioalkyl, Alkylamino, Dialkylamino, Halogenalkyl, Alkenyl, Alkinyl, für gegebenenfalls substituiertes Cycloalkyl oder für jeweils gegebenenfalls substituiertes Aryl, Aryloxy, Arylthio oder Arylamino steht und

$R^7$ für Alkyl, Hydroxyalkyl, Alkoxyalkyl, Halogenalkyl, Alkenyl, Alkinyl, Cycloalkyl oder für jeweils gegebenenfalls substituiertes Aralkyl oder Aryl steht und

Ar für substituiertes Phenyl oder für gegebenenfalls substituiertes Pyridyl steht,

dadurch gekennzeichnet, daß man

(a) 1-Aryl-5-halogen-pyrazole der Formel (II)

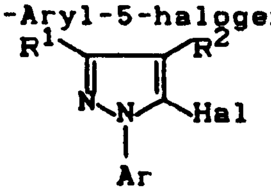

(II)

in welcher

$R^1$, $R^2$ und Ar die oben angegebene Bedeutung haben und

Hal für Halogen steht,

mit Hydrazin-Derivaten der Formel (III)

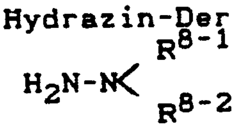
(III)

in welcher

$R^{8-1}$ und $R^{8-2}$ unabhängig voneinander jeweils für Wasserstoff oder für jeweils gegebenenfalls substituiertes Alkyl, Alkenyl, Alkinyl oder Cycloalkyl stehen,

gegebenenfalls in Gegenwart eines Verdünnungsmittels umsetzt, oder daß man

(b) die nach Verfahren (a) erhältlichen 5-Hydrazino-1- aryl-pyrazole der Formel (Ia),

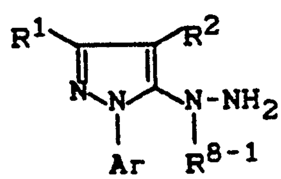
(Ia)

in welcher

$R^1$, $R^2$, $R^{8-1}$ und Ar die oben angegebene Bedeutung haben,

oder der Formel (Ib),

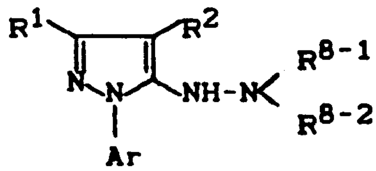

(Ib)

in welcher

$R^1$, $R^2$, $R^{8-1}$, $R^{8-2}$ und Ar die oben angegebene Bedeutung haben,

(α) mit Alkylierungsmitteln der Formel (IV),

$$R^{8-3}-A^1 \qquad (IV)$$

in welcher

$R^{8-3}$ für jeweils gegebenenfalls substituiertes Alkyl, Alkenyl, Alkinyl oder Cycloalkyl steht und

$A^1$ für eine elektronenanziehende Abgangsgruppe steht, oder

(β) mit Acylierungsmitteln der Formel (V),

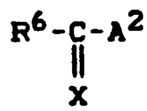

(V)

in welcher

$R^6$ und X die oben angegebene Bedeutung haben und

$A^2$ für eine elektronenanziehende Abgangsgruppe steht,

oder

(γ) mit Iso(thio)cyanaten der Formel (VI)

$$R^{6-1}-N=C=X \qquad (VI)$$

in welcher

$R^{6-1}$ für Alkyl oder für gegebenenfalls substituiertes Aryl steht und

X die oben angegebene Bedeutung hat,

oder

(δ) mit Sulfonylierungsmitteln der Formel (VII),

$$R^7-SO_2-A^3 \qquad (VII)$$

in welcher

$R^7$ die oben angegebene Bedeutung hat und

$A^3$ für eine elektronenanziehende Abgangsgruppe steht,

jeweils gegebenenfalls in Gegenwart eines Verdünnungsmittels sowie gegebenenfalls in Gegenwart eines Säurebindemittels oder Reaktionshilfsmittels umsetzt.

5. Herbizide Mittel, gekennzeichnet durch einen Gehalt an mindestens einem 1-Aryl-5-hydrazino-pyrazol der Formel (I) gemäß den Ansprüchen 1 bis 4.

6. Verfahren zur Bekämpfung von Unkräutern, dadurch gekennzeichnet, daß man 1-Aryl-5-hydrazino-pyrazole der Formel (I) gemäß den Ansprüchen 1 bis 4 auf Unkräuter und/oder ihren Lebensraum einwirken läßt.

7. Verwendung von 1-Aryl-5-hydrazino-pyrazolen der Formel (I) gemäß den Ansprüchen 1 bis 4 zur Bekämpfung von Unkräutern.

8. Verfahren zur Herstellung von herbiziden Mitteln, dadurch gekennzeichnet, daß man 1-Aryl-5-hydrazino-pyrazole der Formel (I) gemäß den Ansprüchen 1 bis 4 mit Streckmitteln und/oder oberflächenaktiven Substanzen vermischt.